# EUROPEAN PATENT APPLICATION

(11) **EP 4 488 364 A1**
(43) Date of publication of application: **08.01.2025**
(21) Application number: 23763772.3
(22) Date of filing: 06.03.2023
(51) Int. Cl.: C12N 5/074, C12N 15/85, A61K 35/545, A61P 37/06

(54) **LOW IMMUNOGENIC STEM CELLS, LOW IMMUNOGENIC CELLS DIFFERENTIATED OR DERIVED FROM STEM CELLS, AND PRODUCTION METHOD THEREFOR**

(30) Priority: 04.03.2022 KR 20220027931
(71) Applicant: Toolgen Incorporated, Seoul 07789 (KR)
(72) Inventor: LEE, Jae Young, Seoul 07789 (KR); LEE, Kang In, Seoul 07789 (KR); SON, Young Ju, Seoul 07789 (KR); GO, Nan Yeong, Seoul 07789 (KR); SHIN, Eun Ji, Seoul 07789 (KR); YU, Hwan Yeul, Seoul 07789 (KR)
(74) Representative: Modiano, Gabriella Diana
(86) International application number: PCT/KR2023/003055
(87) International publication number: WO 2023/167575

(57) **Abstract**

Provided are hypoimmunogenic stem cells, hypoimmunogenic cells differentiated or derived from stem cells capable of inhibiting immune rejection upon transplantation by increasing the expression or activity level of immune activity inhibitory factors, a method of preparing the same, and a cell therapeutic agent using the same.

## Description

### [Technical field]

The present disclosure relates to hypoimmunogenic stem cells capable of inhibiting immune rejection upon transplantation by increasing the expression or activity level of immune activity inhibitory factors, hypoimmunogenic cells differentiated or derived from stem cells, a method of preparing the same, and a cell therapeutic agent using the same.

### [Background Art]

Induced pluripotent stem cells (hereinafter referred to as 'iPSC') are somatic cells isolated from the human body that are dedifferentiated into pluripotent stem cells that can differentiate into all cells in body through artificial stimulation, and it is attracting attention as a regenerative cell therapeutic agent for regenerating damaged cells, tissues, and organs using its pluripotency.

When induced pluripotent stem cells are created from the patient's own cells and differentiated into necessary cells, organoids, organs, and the like and used for the patient's own treatment, the immune rejection is low, so they can be used for autologous cell therapy. However, in the case of such customized stem cell therapy, there are limitations in commercialization because it takes time and manpower to produce cells and tissues for regenerative treatment.

Accordingly, an allogeneic cell therapeutic agent derived from another person, rather than an autologous cell therapeutic agent, which is the patient's own cells, can be an alternative, but it may be recognized by immune cells such as natural killer cells (NK cells) and T cells, which are responsible for important immune mechanisms in the human body, and cause immune rejection.

NK cells bind to their ligands expressed on abnormal cells such as cancer cells and virus-infected cells through various activating receptors and inhibitory receptors on the cell surface, and its activity is regulated by the balance between the induced signals. In addition, the immune system has an inhibitory mechanism called an immune checkpoint to prevent T cells, which can directly attack cells, from being overly activated and attacking normal cells. Activation of T cells begins when the T cell receptor (TCR) present on the cell surface recognizes the antigen bound to the major histocompatibility complex (MHC) molecule of an antigen presenting cell (APC). After the antigen is recognized through TCR-MHC binding, T cells are activated by simultaneously binding CD80 and CD40, which are expressed on APCs, to CD28 and CD40L, which correspond to the ligands on the T cell surface. On the other hand, immune checkpoint receptors such as CTLA-4 and PD-1 on T cells send inhibitory signals that inactivate T cells through binding to immune checkpoint ligands such as CD80, CD86, and PD-L1 on abnormal cells such as cancer cells.

Therefore, genetically engineering the ligands that interact with these immune activation and inhibition-related receptors in vivo may be a desirable alternative to avoid immune rejection.

### [Related Art Documents]

### [Patent Document]

(Patent Document 1) Japanese Patent Publication No. 2018-515139
(Patent Document 2) Korean Patent Publication No. 10-2020-0120939

### [Non-Patent Document]

(Non-Patent Document 1) Nayoung Kim, HunSik Kim, Front. Immunol., 10 September 2018, Targeting Checkpoint Receptors and Molecules for Therapeutic Modulation of Natural Killer Cells
(Non-Patent Document 2) Rupal S. Bhatt et al., November 23, 2020, Cancer Immunology Research. KIR3DL3 is an inhibitory receptor for HHLA2 that mediates an alternative immunoinhibitory pathway to PD1.

### [Technical Problem]

The present disclosure is for the purpose of providing hypoimmunogenic stem cells, hypoimmunogenic cells differentiated or derived from stem cells that may inhibit immune rejection when transplanting cells, tissues, and organs, a method of preparing the same, and a cell therapeutic agent using the same.

### [Technical Solution]

In order to achieve the above object, the present disclosure provides artificially engineered stem cells having artificially engineered genes in which genes encoding one or more immune activity inhibitory factors selected from the group consisting of LGALS9 (Gal-9), CLEC4G (LSECtin), PD-L1, and HHLA2 are inserted into stem cells genomes.

In addition, the present disclosure provides artificially engineered cells differentiated or derived from stem cells, having artificially engineered gene in which genes encoding one or more immune activity inhibitory factors selected from the group consisting of LGALS9 (Gal-9), CLEC4G (LSECtin), PD-L1, and HHLA2 are inserted into genomes of cells differentiated or derived from stem cells.

In addition, the present disclosure provides cells with increase in expression or activity of one or more immune activity inhibitory factors selected from the group consisting of LGALS9 (Gal-9), CLEC4G (LSECtin), PD-L1, and HHLA2, differentiated or derived from the artificially engineered stem cells.

In addition, the present disclosure provides a composition for preparing hypoimmunogenic stem cells or a hypoimmunogenic cells differentiated or derived from stem cells, including: a guide nucleic acid capable of targeting a target sequence located in genomes of stem cells or cells differentiated or derived from stem cells, or a nucleic acid encoding the same; a gene donor encoding one or more immune activity inhibitory factors selected from the group consisting of LGALS9 (Gal-9), CLEC4G (LSECtin), PD-L1, and HHLA2, or a nucleic acid encoding the same; and an editor protein or nucleic acid encoding the same.

The present disclosure also provides a method of preparing hypoimmunogenic stem cells or hypoimmunogenic cells differentiated or derived from stem cells,
including: introducing the composition of preparing hypoimmunogenic stem cells or hypoimmunogenic cells differentiated or derived from stem cells into isolated stem cells or cells differentiated or derived from isolated stem cells; and
knocking in a gene encoding one or more immune activity inhibitory factors selected from the group consisting of LGALS9 (Gal-9), CLEC4G (LSECtin), PD-L1, and HHLA2 into genomes of the stem cells, or genomes of the cells differentiated or derived from stem cells to increase the expression or activity of one or more immune activity inhibitory factors selected from the group consisting of LGALS9 (Gal-9), CLEC4G (LSECtin), PD-L1, and HHLA2 of the isolated stem cells or the cells differentiated or derived from the isolated stem cells.

In addition, the present disclosure provides a composition for cell transplantation or biological tissue regeneration including the artificially engineered stem cells as an active ingredient.

In addition, the present disclosure provides a composition for cell transplantation or biological tissue regeneration including, as an active ingredient, the artificially engineered cells differentiated or derived from stem cells.

In addition, the present disclosure provides a composition for cell transplantation or biological tissue regeneration, including, as an active ingredient, the cells with increase in expression or activity of one or more immune activity inhibitory factors selected from the group consisting of LGALS9 (Gal-9), CLEC4G (LSECtin), PD-L1, and HHLA2, differentiated or derived from the artificially engineered stem cells.

### [Advantageous Effects]

Since the stem cells, or cells differentiated or derived from stem cells of the present disclosure exhibit hypoimmunogenicity to avoid immune rejection when transplanting cells, tissues, and organs immune rejection, the cells can be differentiated into various cells and used as a cell therapeutic agent that does not cause immune rejection in autologous or allogeneic environments.

### [Brief Description of Drawings]

FIG. 1 shows a schematic diagram of inserting three genes, LGALS9 (Gal-9), CLEC4G (LSECtin), and HHLA2 between the left and right homology arms, based on a specific site of AAVS1, one of the safe harbor sites.
FIG. 2 shows the results of PCR reaction from cell DNA and the results of Sanger sequencing using the PCR product obtained above to select single clone from iPSCs prepared in Example 1 in which three genes were knocked-in (KI).
FIG. 3 shows the results of confirming changes in mRNA levels for the three genes knocked-in in Example 1 using real-time PCR.
FIG. 4 is a graph showing the results of confirming the copy number of genes knocked-in (KI) using the ddPCR method in Experimental Example 3.
FIG. 5 shows the results of measuring the HHLA2 expression level of the AAVS1-ALC3#11 clone prepared in Example 1 through flow cytometry in Experimental Example 4.
FIG. 6A shows the results of flow cytometry using an anti-CD31 antibody after differentiating wild-type (WT) iPSCs and iPSCs with knocked-in (KI) three genes prepared in Example 1 into endothelial cells (ECs), and FIG. 6B shows the results confirming that HLA-ABC antigens appear on the surface of endothelial cells (ECs) differentiated from iPSC.

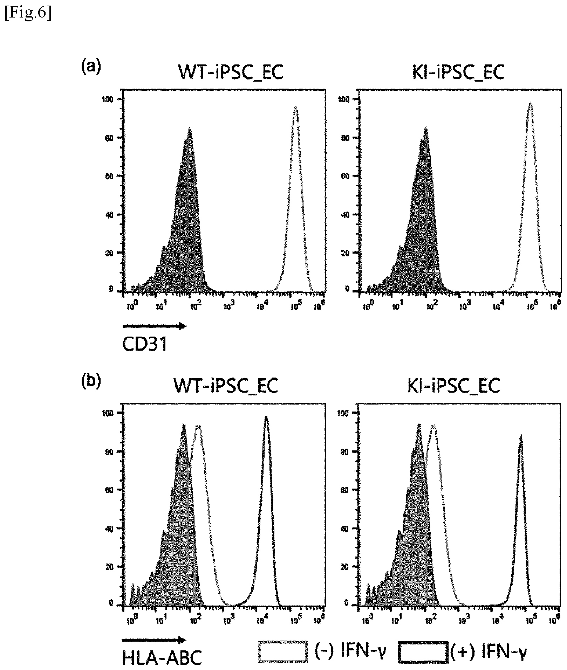

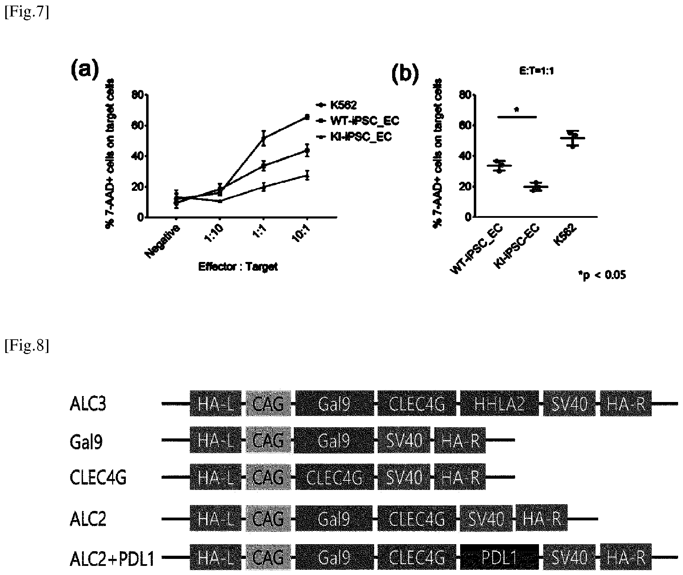

FIG. 7A is a graph showing the results of confirming cytotoxicity by culturing K562 cells, endothelial cells differentiated from wild-type (WT) iPSCs (WT-iPSC_EC), and endothelial cells differentiated from iPSCs in which the three genes prepared in Example 1 were knocked in (KI-iPSC EC) with NK cells, which are effector cells, at various ratios, and FIG. 7B is a graph showing the results of evaluating the cytotoxicity of NK cells in endothelial cells differentiated from wild-type (WT) iPSCs expressing 98% or more of HLA-ABC (WT-iPSC_EC, FIG. 6B) and endothelial cells differentiated from iPSCs in which the three genes prepared in Example 1 were knocked in (KI-iPSC EC).
FIG. 8 is a diagram showing an example of a lentivirus for inserting each gene sequence of LGALS9 (Gal-9)/CLEC4G (LSECtin)/HHLA-2 (SEQ ID NO: 23), LGALS9 (Gal-9) (SEQ ID NO: 24), CLEC4G (LSECtin) (SEQ ID NO: 25), LGALS9 (Gal-9)/CLEC4G (LSECtin) (SEQ ID NO: 26), and LGALS9 (Gal-9)/ CLEC4G (LSECtin)/PD-L1 (SEQ ID NO: 27), and

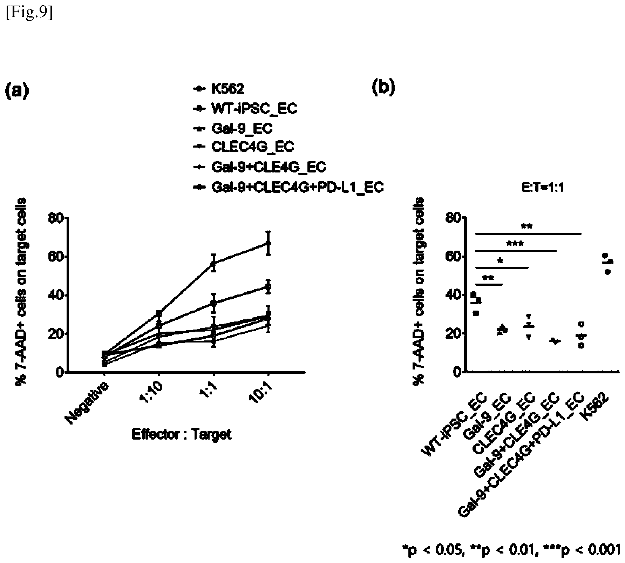

FIG. 9A is a graph showing the results of confirming cytotoxicity by culturing K562 cells, endothelial cells differentiated from wild type (WT) iPSCs (WT-iPSC_EC), endothelial cells in which the Gal-9 gene of SEQ ID NO: 24 was inserted into endothelial cells differentiated from iPSCs (Gal-9_EC), endothelial cells in which the CLEC4G gene of SEQ ID NO: 25 was inserted into endothelial cells differentiated from iPSCs (CLEC4G_EC), endothelial cells in which the Gal-9/CLEC4G gene of SEQ ID NO: 26 was inserted into endothelial cells differentiated from iPSCs (Gal-9+CLEC4G_EC), and endothelial cells in which the Gal-9/CLEC4G/PD-L1 gene of SEQ ID NO: 27 was inserted into endothelial cells differentiated from iPSCs (Gal-9+CLEC4G+ PD-L1_EC) with NK cells, which are effector cells, at various ratios, and FIG. 9B is a graph showing the results of measuring 7-AAD+ cells by co-culturing effector cells (NK cells) and target cells (EC, endothelial cells) at a ratio of 1:1.

### [Best Mode for Implementation of the Invention]

Hereinafter, the present disclosure will be described in more detail.

As used herein, the term "stem cell" refers to a broad concept that collectively refers to undifferentiated cells with stemness, that is, the ability to differentiate into various types of body tissue cells. At this time, the stem cells may be induced pluripotent stem cells, embryonic stem cells, somatic cell nuclear transfer-PSC, or adult stem cells. In addition, the cells may be of human origin, but are not limited thereto.

As used herein, "differentiation" refers to a phenomenon in which cells divide and proliferate and become specialized in their structure or function while the entire organism grows. In other words, the differentiation refers to a process by which cells and tissues of living things change into appropriate forms and functions to perform their assigned roles, and may include, for example, the process by which pluripotent stem cells change into ectoderm (cerebral cortex, midbrain, hypothalamus, etc.), mesoderm (yolk sac, etc.), and endoderm cells, as well as the process by which hematopoietic stem cells change into red blood cells, white blood cells, platelets, and the like, namely, the process by which progenitor cells express specific differentiation traits.

As used herein, "expression increase" means showing a higher level of expression than the expression level of protein measured in the wild-type. The increase may be about 5% or more, about 10% or more, about 15% or more, about 20% or more, about 30% or more, about 50% or more, about 60% or more, about 70% or more, or about 100% or more compared to non-genetically modified cells or wild-type cells. The increase in expression may be result from an increase in gene copy number or enhanced transcription or elevated translation.

As used herein, the term "increase in activity" or "increased activity" may refer to a detectable rise in protein or enzyme activity. "Increase in activity" or "increased activity" means a higher level of protein or enzyme activity compared to a given parental or wild-type cells.

As used herein, an "immune activity inhibitory factor" refers to a ligand that may inhibit immune cell activity by specifically binding to an immune checkpoint receptor or an immunosuppressive receptor (inhibitory immune receptor) expressed in immune cells such as T cells and NK cells.

As used herein, "hypoimmunogenicity" means that the cells demonstrating reduced or eliminated immune rejection when transplanted to another individual relative to the wild type. The reduction in immune rejection may be 20% or more, about 30% or more, about 40% or more, about 50% or more, about 55% or more, about 60% or more, about 70% or more, about 75% or more, about 80% or more, about 85% or more, about 90% or more, about 95% or more, or about 100%.

Provided are hypoimmunogenic stem cells or hypoimmunogenic cells differentiated or derived from stem cells, which are capable of inhibiting immune rejection upon transplantation by increasing the expression or activity level of immune inhibitory factors.

The stem cells exhibit increased expression or activity of a ligand that binds to an immune checkpoint receptor which inhibits immune cell activation of T cells and NK cells, and show enhanced expression or activity of a ligand that binds to an immunosuppressive receptor (inhibitory immune receptor), and when used as a cell therapeutic agent in an autologous or allogeneic environment, the immune rejection can be suppressed and a state of immune tolerance may be achieved.

In one embodiment of the present invention, the stem cells, or cells differentiated or derived from stem cells may exhibit increased expression or activity of one or more immune suppression factors selected from the group consisting of LGALS9 (Gal-9), CLEC4G (LSECtin), PD-L1, and HHLA2 compared to wild-type stem cells or wild-type cells.

Galectin-9 (Gal-9) or LGALS9 is a known ligand for T-cell immunoglobulin and mucin domain protein 3 (TIM-3), and known by many different names such as lectin, galactoside-binding, soluble, 9; tumor antigen HOM-HD-21; Ecalectin; Gal-9; urate transporter/channel protein; LGALS9A; and HUAT. TIM-3 is an immunosuppressive receptor, expressed in T cells, regulatory T cells, B cells, dendritic cells, NK cells, and macrophages, and when TIM-3 binds to galectin-9, its ligand, the activity of T cells and NK cells is inhibited.

Liver and lymph node sinusoidal endothelial cell C-type lectin (LSECtin) is a ligand for LAG-3 (lymphocyte activation gene 3). LAG-3 is one of several immune checkpoint proteins such as TIM3, CTLA-4, PD-1, and PD-L1, and is expressed in helper T cells, cytotoxic T cells, regulatory T cells, some NK cells, and B cells. Immune evasion occurs when immune checkpoint proteins located on the surface of T cells, and specific ligands present in abnormal cells combine to neutralize T cells and NK cells.

Programmed cell death ligand-1 (PD-L1) is a transmembrane protein present in normal cells, and binds to PD-1 to induce evasion of immune attack as a ligand for PD-1 that binds to PD-1 on T cells. Meanwhile, in order for T cells to be activated, in addition to the primary signal stimulation of the T cell receptor and antigen, secondary signal stimulation (costimulation) is simultaneously required. At this time, when either signal is missing, the T cell becomes inactive. Programmed death-1 (PD-1) is a secondary signal stimulating factor (immune check point or immune modulator) that regulates the secondary signal activity of T cells, and may act to inhibit T cell function, such as inhibiting T cell proliferation and reducing cytokine expression by binding to PD-L1 or B7.1 (CD80) expressed on the surface of cells such as activated T cells (CD8 and/or CD4) or dendritic cells.

Human endogenous retrovirus-H long terminal repeat associating protein 2 (HHLA2) is a newly identified B7 family member that regulates T cell function. HHLA2 has been identified as a specific ligand for transmembrane and immunoglobulin domain containing 2 (TMIGD2), and HHLA2/TMIGD2 interaction selectively co-stimulates human T-cell growth and cytokine production through an AKT-dependent signaling cascade. In addition, HHLA2 binds to killer cell immunoglobulin-like receptor, three immunoglobulin domains and long cytoplasmic tail 3 (KIR3DL3), a receptor on T and NK cells, and also inhibits T cell activation.

According to one embodiment of the present invention, the stem cells are artificially engineered stem cells having artificially engineered genes in which genes encoding one or more immune activity inhibitory factors selected from the group consisting of LGALS9 (Gal-9), CLEC4G (LSECtin), PD-L1, and HHLA2 are inserted into stem cells genomes.

In one embodiment of the present invention, the cells differentiated or derived from stem cells are artificially engineered cells differentiated or derived from stem cells, having artificially engineered genes in which genes encoding one or more immune activity inhibitory factors selected from the group consisting of LGALS9 (Gal-9), CLEC4G (LSECtin), PD-L1, and HHLA2 are inserted into genomes of cells differentiated or derived from stem cells.

In one embodiment of the present invention, the cells differentiated or derived from stem cells are one selected from the group consisting of human vascular endothelial cells, pancreatic cells, osteocytes, chondrocytes, cardiomyocytes, muscle cells, epidermal cells, fibroblasts, nerve cells, hepatocytes, enterocytes, stomach cells, skin cells, fat cells, blood cells, immune cells, cell spheroids, and organoids.

In one embodiment of the present invention, the gene sequence of the artificially engineered stem cells differs from the gene sequence of the wild-type stem cells, and may exhibit increased expression or activity of a gene encoding one or more immune activity inhibitory factors selected from the group consisting of LGALS9 (Gal-9), CLEC4G (LSECtin), PD-L1, and HHLA2, or one or more immune activity inhibitory factors selected from the group consisting of LGALS9 (Gal-9), CLEC4G (LSECtin), PD-L1, and HHLA2, compared to the wild-type stem cells.

According to one embodiment of the present invention, the gene sequence of artificially engineered cells differentiated or derived from stem cells differs from the gene sequence of the wild-type cells, and may exhibit increased expression or activity of a gene encoding one or more immune activity inhibitory factors selected from the group consisting of LGALS9 (Gal-9), CLEC4G (LSECtin), PD-L1, and HHLA2, or one or more immune activity inhibitory factors selected from the group consisting of LGALS9 (Gal-9), CLEC4G (LSECtin), PD-L1, and HHLA2, compared to the wild-type cells.

As used herein, "increase in expression or activity" may refer to the protein being expressed at a higher level compared to wild-type stem cells or wild-type cells, or exhibiting high activity even when the protein is expressed.

That is, in stem cells, or cells differentiated or derived from stem cells, the expression or activity of one or more immune activity inhibitory factors selected from the group consisting of LGALS9 (Gal-9), CLEC4G (LSECtin), PD-L1, and HHLA2 may be increased by about 20% or more, about 30% or more, about 40% or more, about 50% or more, about 55% or more, about 60% or more, about 70% or more, about 75% or more, about 80% or more, about 85% or more, about 90% or more, about 95% or more, or about 100%, compared to the expression or activity of wild-type stem cells or wild-type cells.

In the present disclosure, the artificial modification of a gene nucleic acid sequence may be accomplished by altering the nucleic acids that constitute the gene. This may include mutations, substitutions, or deletions of portions or entire genes, or the insertion of one or more bases into the gene, and utilizing gene editing technologies. As an example, the artificial modification may be implanted though non-homologous end joining (NHEJ) or homology directed repair (HDR) mechanisms.

In one embodiment of the present invention, the stem cells, or cells differentiated or derived from stem cells may have genes knocked in with a gene encoding one or more immune activity inhibitory factors selected from the group consisting of LGALS9 (Gal-9), CLEC4G (LSECtin), PD-L1, and HHLA2.

As used herein, "knock-in" refers to the insertion of a specific nucleic acid or gene into a target gene or nucleic acid within an animal cell's genome.

Cells possess unique mechanisms to repair any double-stranded or single-stranded DNA damage. DNA double-strand break (DSB) repair mechanisms are categorized into non-homologous end joining (NHEJ) and homologous recombination (HR).

Homology directed repair (HDR) is a method that uses a homologous sequence as a template to repair or restore damaged genes or nucleic acids, ensuring error-free correction. Typically, in order to repair or restore broken DNA, this utilize either an information on an unmodified complementary nucleotide sequence or information on a sister chromatid is used to repair or restore the broken DNA.

To repair or restore damaged DNA using HDR, instead of using the complementary nucleotide sequence or sister chromatids that the cell originally possesses, broken DNA may be repaired or restored by providing, to the cell, a DNA template artificially synthesized using complementary nucleotide sequence or homologous nucleotide sequence information (that is, a nucleic acid template including complementary nucleotide sequence or homologous nucleotide sequence). At this time, when repairing or repairing the broken DNA by including a nucleic acid sequence or nucleic acid fragment in addition to the nucleic acid template, nucleic acid sequences or nucleic acid fragments additionally included in the broken DNA may be knocked-in.

For an example, using the CRISPR complex to cleave either the double strand or single strand of the target gene or nucleic acid, and then providing a cell with a nucleic acid template that includes a nucleotide sequence complementary to the nucleotide sequence adjacent to the cleavage site, allows the cleaved nucleotide sequence of the target gene or nucleic acid to be repaired or restored via HDR method. Homology-independent targeted integration (HITI) enables the insertion of transgenes in proliferating and non-proliferating cells (Suzuki et al., Nature, 2016, 540(7631):144 -149). HITI employs CRISPR/Cas9 to target the insertion site and provides a donor that includes a specific nucleotide sequence for insertion between the cleaved target DNA ends through non-homologous end joining (NHEJ).

As an example, CRISPR/Cas9 may be used to cleave the target gene and knock-in a donor including a specific nucleotide sequence via the HITI method.

In one embodiment of the present invention, a gene encoding one or more immune activity inhibitory factors selected from the group consisting of LGALS9 (Gal-9), CLEC4G (LSECtin), PD-L1, and HHLA2 may be inserted into a safe harbor site in the genome of stem cells, or genomes of cells differentiated or derived from stem cells, but is not limited thereto. In an example embodiment, the gene encoding the immune activity inhibitory factor may be inserted into the AAVS1 locus, CCR5 locus, CLYBL locus, ROSA26 locus, and SHS231 locus (chromosome 4 'safe harbor site' 231) of the genome.

The present disclosure provides a method of preparing hypoimmunogenic stem cells, or hypoimmunogenic cells differentiated or derived from stem cells, including: increasing the expression or activity of one or more immune activity inhibitory factors selected from the group consisting of LGALS9 (Gal-9), CLEC4G (LSECtin), PD-L1, and HHLA2 of stem cell, or cells differentiated or derived from stem cells.

The increase in expression or activity of the immune activity inhibitory factor/protein may be achieved by artificial modification of the corresponding genes, for example, using gene editing technology.

For an example, the gene editing technology may include but is not limited to, TALEN (transcription activator-like effector nuclease) which fuses a TAL effector (transcription activator-like effector or TALE) domain and a cleavage domain, zinc-finger nuclease, or CRISPR enzyme system derived from the microbial immune system CRISPR.

"TALEN" refers to a nuclease capable of recognizing and cleaving a target region of DNA. The TALEN is a fusion protein including a TALE domain and a nucleotide cleavage domain. A TAL effector are known to be a protein secreted through the type secretion system of Xanthomonas bacteria when they are infected various plant species. The protein may bind to a promoter sequence in the host plant to activate the expression of plant genes that aid bacterial infection. The protein recognizes a plant DNA sequence through a central repeat domain consisting of a variable number of amino acid repeats up to 34. A "TALE domain" or "TALE" is a polypeptide comprising one or more TALE repeat domains/units. This repeat domain is involved in the binding of the TALE to its cognate target DNA sequence. A single "repeat unit" is typically 33-35 amino acids in length and exhibits at least some sequence homology to other TALE repeat sequences within naturally occurring TALE proteins. The TALE domain is a protein domain that binds to nucleotides in a sequence-specific manner through one or more TALE-repeat modules.

The TALE DNA binding domain includes, but is not limited to, at least one TALE-repeat module, more specifically 1 to 30 TALE-repeat modules. The TALE DNA binding domain may comprise half of a TALE-repeat module. The entire contents disclosed in International Patent Publication No. WO2012/093833 or U.S. Patent Publication No. 2013-0217131 with respect to the above TALE are incorporated in the present specification by reference.

"Zinc finger DNA binding protein" (or binding domain) is a protein or a domain within a larger protein that binds DNA in a sequence-specific manner through one or more zinc fingers, which are regions of the amino acid sequence within the binding domain whose structure is stabilized through coordination of zinc ions. The term "zinc finger DNA binding protein" is commonly abbreviated as zinc finger protein or ZFP.

The zinc-finger nuclease (ZFN) includes a zinc-finger protein engineered to bind to a target site of a target gene and a cleavage domain or a cleavage half-domain. The ZFN may be an artificial restriction enzyme including a zinc-finger DNA binding domain and a DNA cleavage domain. Here, the zinc-finger DNA binding domain may be engineered to bind to the selected sequence. For example, Beerli et al. (2002) Nature Biotechnol. 20:135-141; Pabo et al. (2001) Ann. Rev. Biochem. 70:313-340; Isalan et al, (2001) Nature Biotechnol. 19: 656-660; Segal et al. (2001) Curr. Opin. Biotechnol. 12:632-637; and Choo et al. (2000) Curr. Opin. Struct. Biol.10:411-416 may be included as references in the present specification. Compared to naturally occurring zinc finger proteins, engineered zinc finger binding domains may have novel binding specificities. An engineering method includes, but are not limited to, rational design and selection of various types. The rational design includes the use of databases including, for example, triple (or quadruple) nucleotide sequences, and individual zinc finger amino acid sequences, and at this time, each triple or quadruple nucleotide sequence is associated with one or more sequences of zinc fingers that bind to a specific triple or quadruple sequence.

Selection of target genes and target sequences in the ZFN, design and construction of fusion proteins or polynucleotides encoding them are known to those skilled in the art, which is described in detail in the full text of U.S. Patent Nos. 7,888,121 and 8,409,861 as references, and the entirety of the published patents is incorporated herein by reference. In addition, as disclosed in these references and others in the art, zinc finger domains and/or multi-finger zinc finger proteins may be linked together by any suitable linker sequence, for example a linker comprising a linker of 5 or more amino acids in length.

Linker sequences, at least six or more amino acids in length, are exemplified in U.S. Registered Patent Nos. 6,479,626; 6,903,185; and 7,153,949. The proteins described herein may include any combination of suitable linkers between each zinc finger of the protein.

Nucleases such as the ZFNs include a cleavage domain and/or a cleavage half-domain that is the nuclease active portion. As an example, the cleavage domain may be a cleavage domain derived from a nuclease different from the zinc-finger DNA binding domain, that is, a heterologous cleavage domain.

As another example, the cleavage half-domain may be a fusion protein derived from any nuclease or part thereof that requires dimerization for cleavage activity. When the fusion protein includes a cleavage half-domain, generally two fusion proteins may be required for cleavage. Alternatively, a single protein including two cleavage half-domains may be used. The two cleavage half-domains may be derived from the same endonuclease (or functional fragments thereof), or each cleavage half-domain may be derived from a different endonuclease (or functional fragments thereof). In addition, the target site of the two fusion proteins is preferably positioned so that the cleavage half-domains may form a functional cleavage domain, for example by dimerization by positioning the cleavage half-domains in a spatial orientation with respect to each other through the binding of two fusion proteins to their respective target sites.

The "CRISPR-enzyme system" consists of a guide nucleic acid and/or an editor protein. "Guide nucleic acid" refers to a nucleic acid capable of recognizing a target nucleic acid, target gene, or target chromosome and interacting with an editor protein. At this time, the guide nucleic acid may form a complementary bond with some nucleotides in the target nucleic acid, target gene, or target chromosome. The guide nucleic acid may be in the form of a target DNA-specific guide RNA, DNA encoding the guide RNA, or a DNA/RNA mixture.

The guide nucleic acid may be a guide RNA. The "guide RNA" may be transcribed in vitro, but is not limited to, particularly from an oligonucleotide double strand, or a plasmid template.

The design and composition of the guide RNA are known to those skilled in the art and are described in detail in Korean Registration Patents 10-1656236, 10-1656237, 10-1706085, 10-2052286, and 10-2182847, and the entirety of the above Registration Patents is incorporated herein as reference material for the present disclosure.

The guide nucleic acid may include a scaffold sequence portion and a guide sequence portion. The scaffold sequence portion is a portion that interacts with the Cas protein and allows the Cas protein and a guide nucleic acid to bind to form a complex (ribonucleoprotein, RNP). Generally, the scaffold sequence portion includes tracrRNA and some sequence portions of crRNA, and the scaffold sequence is determined depending on which Cas protein is used.

The guide sequence portion is a nucleotide sequence portion that may bind complementarily to some sequences of any one of the double strands of a target gene or nucleic acid, and a nucleotide sequence portion that may be artificially modified, and is determined by the target nucleotide sequence of interest. At this time, the guide sequence may be a nucleotide sequence having at least 50%, 55%, 60%, 65%, 70%, 75%, 80%, 85%, 90%, or 95% or more complementarity or complete complementarity with the guide nucleic acid binding sequence of the target gene or target nucleic acid. The guide sequence may be a sequence included in the guide domain of the guide nucleic acid.

The guide sequence portion may be included in crRNA. As an example, the guide nucleic acid may be a dual RNA including two RNAs, namely, crRNA (CRISPR RNA) and tracrRNA (trans-activating crRNA) as components.

As another example, the guide nucleic acid may be a single-chain guide RNA (sgRNA) which links the main portions of crRNA and tracrRNA.

The target sequence may be a nucleotide sequence of a certain length present in the target gene or target nucleic acid, and specifically, some nucleotide sequences within the target region classified by a regulatory region of the target gene, coding region (or CDS, coding sequence), or a non-coding region (or untranslated region (UTR)), or one or more some nucleotide sequences selected from a combination of the target regions. The target sequence may be a target of a guide nucleic acid-editor protein complex (RNP).

In one embodiment of the present invention, the target sequence may be a safe harbor site, but is not limited thereto. In an example embodiment, the safe harbor site may be one selected from the group consisting of AAVS1 locus, CCR5 locus, CLYBL locus, ROSA26 locus, and the SHS231 locus.

The target sequence is a nucleotide sequence adjacent to a protospacer-adjacent motif (PAM) sequence recognized by the editor protein, and may include all or part of the PAM, but is not limited thereto.

The term target sequence may be used to mean both types of nucleotide sequence information. For example, in the case of a target gene, the target sequence may mean sequence information of the transcribed strand of the target gene DNA, or may mean nucleotide sequence information of the non-transcribed strand.

The target sequence includes a guide nucleic acid binding sequence or a guide nucleic acid non-binding sequence. Guide nucleic acid binding sequence is a nucleotide sequence that has partial or complete complementarity with the guide sequence included in the guide domain of a guide nucleic acid, and may bind complementary to the guide sequence included in the guide domain of the guide nucleic acid. The target sequence and guide nucleic acid binding sequence are nucleotide sequences that may vary depending on the target gene or nucleic acid, that is, depending on the subject to be genetically engineered or corrected, and the guide nucleic acid may be designed in various ways depending on the target gene or target nucleic acid.

The guide nucleic acid non-binding sequence is a nucleotide sequence that has partial or complete complementarity with the guide sequence included in the guide domain of the guide nucleic acid, and cannot bind complementary to the guide sequence included in the guide domain of the guide nucleic acid. In addition, the guide nucleic acid non-binding sequence is a nucleotide sequence that is complementary to the guide nucleic acid binding sequence and may bind complementary to the guide nucleic acid binding sequence. The guide nucleic acid binding sequence is some nucleotide sequences of the target sequence, and may be a nucleotide sequence having two different sequence orders of the target sequence, that is, one nucleotide sequence of two nucleotide sequences capable of complementary binding to each other. At this time, the guide nucleic acid non-binding sequence may be the remaining nucleotide sequence of the target sequence excluding the guide nucleic acid binding sequence.

The guide nucleic acid binding sequence may be a target sequence, that is, one nucleotide sequence selected from a nucleotide sequence identical to the transcribed strand and a nucleotide sequence identical to the non-transcribed strand. At this time, the guide nucleic acid non-binding sequence may be the guide nucleic acid binding sequence among the target sequences, that is, the remaining nucleotide sequence excluding one nucleotide sequence selected from the nucleotide sequence identical to the transcribed strand and the nucleotide sequence identical to the non-transcribed strand.

The guide nucleic acid binding sequence may be the same length as the target sequence. The guide nucleic acid non-binding sequence may be the same length as the target sequence or guide nucleic acid binding sequence. The guide nucleic acid binding sequence may be 5 to 50 nucleotide sequences.

In one embodiment, the guide nucleic acid binding sequence is 16 nucleotide sequences, 17 nucleotide sequences, 18 nucleotide sequences, 19 nucleotide sequences, 20 nucleotide sequences, 21 nucleotide sequences, 22 nucleotide sequences, 23 nucleotide sequences, 24 nucleotide sequences, or 25 nucleotide sequences. The guide nucleic acid non-binding sequence may be 5 to 50 nucleotide sequences.

In one embodiment, the guide nucleic acid non-binding sequence is 16 nucleotide sequences, 17 nucleotide sequences, 18 nucleotide sequences, 19 nucleotide sequences, 20 nucleotide sequences, 21 nucleotide sequences, 22 nucleotide sequences, 23 nucleotide sequences, 24 nucleotide sequences, or 25 nucleotide sequences.

The guide nucleic acid binding sequence may form a partial or complete complementary bond with the guide sequence included in the guide domain of the guide nucleic acid, and the length of the guide nucleic acid binding sequence may be the same as the length of the guide sequence.

The guide nucleic acid binding sequence may be a nucleotide sequence complementary to the guide sequence included in the guide domain of the guide nucleic acid, for example, a nucleotide sequence that is at least 70%, 75%, 80%, 85%, 90%, or 95% complementary or completely complementary.

As an example, the guide nucleic acid binding sequence may have or include a 1 to 8 nucleotide sequences that is not complementary to the guide sequence included in the guide domain of the guide nucleic acid.

The guide nucleic acid non-binding sequence may have partial or complete homology with the guide sequence included in the guide domain of the guide nucleic acid, and the length of the guide nucleic acid non-binding sequence may be the same as the length of the guide sequence. As an example, the guide sequence may be designed based on a sequence having homology with the guide nucleic acid non-binding sequence.

The guide nucleic acid non-binding sequence may be a nucleotide sequence having homology with the guide sequence included in the guide domain of the guide nucleic acid, for example, having at least 70%, 75%, 80%, 85%, 90%, or 95% homology, or it may be completely homologous.

As an example, the guide nucleic acid non-binding sequence may have or include 1 to 8 nucleotide sequences that are not homologous to the guide sequence contained in the guide domain of the guide nucleic acid. The guide nucleic acid non-binding sequence may bind complementarily to the guide nucleic acid binding sequence, and the guide nucleic acid non-binding sequence may be the same length as the guide nucleic acid binding sequence.

The guide nucleic acid non-binding sequence may be a nucleotide sequence complementary to the guide nucleic acid binding sequence, for example, having at least 90% or 95% complementary or it may be completely complementary nucleotide sequence.

As an example, the guide nucleic acid non-binding sequence may have or include 1 to 2 nucleotide sequences that are not complementary to the guide nucleic acid binding sequence. In addition, the guide nucleic acid binding sequence may be a nucleotide sequence located close to a complementary sequence complementary to a nucleotide sequence (PAM sequence) that may be recognized by the editor protein.

As an example, the guide nucleic acid binding sequence may be a contiguous 5 to 50 nucleotide sequences located adjacent to the 5' end or/and 3' end of a sequence complementary to a nucleotide sequence (PAM sequence) that may be recognized by the editor protein.

In addition, the guide nucleic acid non-binding sequence may be a nucleotide sequence located close to a nucleotide sequence (PAM sequence) that may be recognized by the editor protein.

As an example, the guide nucleic acid non-binding sequence may be a contiguous 5 to 50 nucleotide sequences located adjacent to the 5' end or/and 3' end of a nucleotide sequence (PAM sequence) that may be recognized by the editor protein.

"Editor protein" means a peptide, polypeptide or protein that either bind directly to nucleic acids, or do not bind directly, but may interact a nucleic acid. The editor protein is conceptually referred to as "artificially engineered nuclease" or RNA-guided endonuclease RGEN).

In one embodiment, the editor protein may be a CRISPR enzyme. "CRISPR enzyme" is the main protein component of the CRISPR-enzyme system, also called "Cas protein (CRISPR associated protein)", and refers to a nuclease that may recognize a target sequence and cleave DNA by mixing or forming a complex with a guide RNA.

CRISPR enzymes are known to those skilled in the art, see Korean Registration Patents Nos. 10-1656236, 10-1656237, 10-1706085, 10-2052286, and 10-2182847. The CRISPR enzyme is used herein as a concept that includes all variants that may act as an endonuclease or nickase activated in cooperation with a guide RNA, in addition to the native protein. In the case of activated endonuclease or nickase, target DNA may be cut and this may be used to perform genome editing. In addition, in the case of an inactivated variant, the genome editing may be used to regulate transcription or isolate the desired DNA.

The CRISPR enzyme is a nucleic acid or polypeptide (or protein) having a sequence encoding the CRISPR enzyme, typically, Type II CRISPR enzyme or Type V CRISPR enzyme is widely used, and the Type II CRISPR enzyme is CRISPR associated protein 9 (Cas9) protein.

The Cas9 protein may be derived from various microorganisms such as *Streptococcus pyogenes, Streptococcus thermophilus, Streptococcus sp, Streptomyces pristinaespiralis, Streptomyces viridochromogenes, Streptosporangium roseum, Streptosporangium roseum, Campylobacter jejuni, Staphylococcus Auricularis.*

In order for the Cas9 protein to induce a double-stranded DNA break, the Cas9 protein recognizes a protospacer-adjacent motif (PAM) sequence, which is a nucleotide sequence of a certain length, and requires that a portion of the guide RNA (the guide sequence portion) bind complementary to the complementary strand (the guide nucleic acid binding sequence) of a single strand of DNA (the guide nucleic acid non-binding sequence) on which the target sequence is located.

This PAM sequence is a sequence determined depending on the type or origin of the Cas9 protein, for example, a *Streptococcus pyogenes-derived* Cas9 protein (SpCas9) may recognize the 5'-NGG-3' sequence (complementary sequence: 5'-CCN-3') in the target nucleic acid. At this time, N is one of adenosine (A), thymidine (T), cytidine (C), and guanosine (G).

In addition, the Type V CRISPR enzyme includes Cpf1, and Cpf1 may be *Streptococcus, Campylobacter, Nitratifractor, Staphylococcus, Parvibaculum, Roseburia*, *Neisseria, Gluconacetobacter, Azospirillum*, *Sphaerochaeta*,*Lactobacillus*, *Eubacterium, Corynebacter, Carnobacterium, Rhodobacter, Listeria, Paludibacter, Clostridium, Lachnospiraceae, Clostridiaridium, Leptotrichia*, *Francisella, Legionella, Alicyclobacillus*, *Methanomethyophilus, Porphyromonas, Prevotella, Bacteroidetes, Helcococcus, Letospira*, *Desulfovibrio, Desulfonatronum, Opitutaceae, uberibacillus, Bacillus, Brevibacilus, Methylobacterium,* or *Acidaminococcus*-derived Cpf1.

CRISPR enzymes such as the Cas9 or Cpf1 protein may be isolated from microorganisms present in nature or produced unnaturally through recombinant or synthetic methods. The Cas protein may also be in a form that is easy to introduce into cells. For example, the Cas protein may be linked to a cell-penetrating peptide or protein transduction domain. The protein transduction domain may be poly-arginine or HIV-derived TAT protein, but is not limited thereto. Since various types of cell-penetrating peptides or protein transduction domains are known in the art in addition to the examples described above, those skilled in the art are not limited to the above examples and may apply various examples to the present specification. In addition, the Cas protein may be fused with a functional domain such as a nuclear localization sequence or signal (NLS).

The present disclosure provides a composition for preparing hypoimmunogenic stem cells or hypoimmunogenic cells differentiated or derived from stem cells, including: a guide nucleic acid capable of targeting a target sequence located in genomes of stem cells or cells differentiated or derived from stem cells, or a nucleic acid encoding the same; a gene donor encoding one or more immune activity inhibitory factors selected from the group consisting of LGALS9 (Gal-9), CLEC4G (LSECtin), PD-L1, and HHLA2, or a nucleic acid encoding the same; and an editor protein or nucleic acid encoding the same.

In one embodiment of the present invention, the target sequence may be a safe harbor site, but is not limited thereto. In an example embodiment, the safe harbor site may be one selected from the group consisting of AAVS1 locus, CCR5 locus, CLYBL locus, ROSA26 locus, and SHS231 locus.

In addition, the composition for gene modification may optionally further include a donor including a specific nucleotide sequence to be inserted or a nucleic acid encoding the same.

The donor refers to exogenous nucleotide sequences that may express a specific peptide or protein, and may be inserted into genomic DNA via homology directed repair (HDR). In this case, the inserted nucleic acid sequence may be a partial nucleotide sequence of a gene encoding one or more immune activity inhibitory factors selected from the group consisting of LGALS9 (Gal-9), CLEC4G (LSECtin), PD-L1, and HHLA2.

The donor may be a double-stranded nucleic acid or a single-stranded nucleic acid. The donor may be linear or circular.

The donor may be in the form of a viral vector or a non-viral vector (for example, a plasmid). The virus may be a DNA virus or an RNA virus. At this time, the DNA virus may be a double-stranded DNA (dsDNA) virus or a single-stranded DNA (ssDNA) virus. At this time, the RNA virus may be a single-stranded RNA (ssRNA) virus. The viral vector may be one or more viral vectors selected from the group consisting of retrovirus, lentivirus, adenovirus, adeno-associated virus (AAV), vaccinia virus, poxvirus, and herpes simplex virus (HSV).

The target sequence may be a target of a guide nucleic acid-editor protein complex, and the target sequence may include a protospacer-adjacent motif (PAM) sequence recognized by the editor protein, but is not limited thereto.

In the present specification, the guide nucleic acid, editor protein or guide nucleic acid-editor protein complex and/or donor may be delivered or introduced into the subject in various forms.

In this case, the "subject" refers to an organism into which the guide nucleic acid, editor protein, or guide nucleic acid-editor protein complex is introduced; an organism in which a guide nucleic acid, an editor protein, or a guide nucleic acid-editor protein complex operates; or a specimen or sample obtained from the organism.

The subject may be an organism including a target gene, a target nucleic acid, or a target chromosome of the guide nucleic acid-editor protein complex.

The organism may be an animal, animal tissue, or animal cell. At this time, the tissue may be the eye, skin, liver, kidney, heart, lung, brain, muscle, or blood.

The cells may be stem cells, liver cells, cardiac muscle cells, endothelial cells, or pancreatic cells. The specimen or sample may be acquired from an organism including target gene, target nucleic acid, or target chromosome, such as saliva, blood, liver tissues, brain tissues, liver cells, nerve cells, phagocytes, macrophages, T cells, B cells, astrocytes, cancer cells, or stem cells. The guide nucleic acid, editor protein, or guide nucleic acid-editor protein complex may be delivered or introduced into the subject in the form of DNA, RNA, or a mixture thereof.

At this time, DNA, RNA, or a mixture thereof encoding the guide nucleic acid and/or the editor protein may be delivered or introduced into the subject by methods known in the art. Alternatively, the form of DNA, RNA, or a mixture thereof encoding the guide nucleic acid and/or the editor protein may be delivered or introduced into the subject via vector, non-vector, or a combination thereof.

The vector may be a viral vector or a non-viral vector (for example, a plasmid). The virus may be a DNA virus or an RNA virus. At this time, the DNA virus may be a double-stranded DNA (dsDNA) virus or a single-stranded DNA (ssDNA) virus. At this time, the RNA virus may be a single-stranded RNA (ssRNA) virus. The viral vector may be one or more selected from the group consisting of retrovirus, lentivirus, adenovirus, adeno-associated virus (AAV), vaccinia virus, poxvirus, and herpes simplex virus.

The non-vector may be naked DNA, DNA complex, or mRNA.

In one embodiment of the present disclosure, the nucleic acid sequence encoding the guide nucleic acid and/or the editor protein may be delivered or introduced into the subject in the form of one or more vectors.

The vector may include a guide nucleic acid and/or a nucleic acid sequence encoding an editor protein. As an example, the vector may simultaneously include a guide nucleic acid and a nucleic acid sequence encoding an editor protein. As another example, the vector may include a nucleic acid sequence encoding a guide nucleic acid. For example, the nucleic acid sequence encoding the guide nucleic acid may be all included in one vector, or the nucleic acid sequence encoding the guide nucleic acid may be divided and included in multiple vectors. As another example, the vector may include a nucleic acid sequence encoding an editor protein. For example, in the case of the editor protein, the nucleic acid sequence encoding the editor protein may be included in one vector, or the nucleic acid sequence encoding the editor protein may be divided and included in multiple vectors.

The editor protein may be delivered or introduced into the subject in the form of peptide, polypeptide, or protein.

The editor protein may be delivered or introduced into the subject in the form of peptide, polypeptide, or protein by methods known in the art.

The guide nucleic acid and the editor protein may be delivered or introduced into the subject in the form of a nucleic acid-protein mixture.

The guide nucleic acid and the editor protein may be delivered or introduced into the subject in the form of a guide nucleic acid-editor protein complex. For example, the guide nucleic acid may be DNA, RNA, or a mixture thereof. The editor protein may be in the form of peptide, polypeptide, or protein. As an example, in the case of the guide nucleic acid and the editor protein, a guide nucleic acid in the form of RNA and an editor protein in the form of a protein may be delivered or introduced into the subject in the form of a guide nucleic acid-editor protein complex, that is, ribonucleoprotein (RNP). In addition, the present disclosure provides a method of preparing hypoimmunogenic stem cells, including: contacting stem cells with the composition of preparing hypoimmunogenic stem cells to edit a gene encoding one or more immune activity inhibitory factors selected from the group consisting of LGALS9 (Gal-9), CLEC4G (LSECtin), PD-L1, and HHLA2 to increase the expression or activity of one or more immune activity inhibitory factors selected from the group consisting of LGALS9 (Gal-9), CLEC4G (LSECtin), PD-L1, and HHLA2 of stem cells.

In one embodiment of the present invention, the method of preparing hypoimmunogenic stem cells or hypoimmunogenic cells differentiated or derived from stem cells may include: introducing the composition of preparing hypoimmunogenic stem cells or hypoimmunogenic cells differentiated or derived from stem cells into isolated stem cells or cells differentiated or derived from the isolated stem cells; and

knocking in a gene encoding one or more immune activity inhibitory factors selected from the group consisting of LGALS9 (Gal-9), CLEC4G (LSECtin), PD-L1, and HHLA2 into genomes of stem cells, or genomes of the cells differentiated or derived from the stem cells into genomes of the stem cells, or the cells differentiated or derived from stem cells to increase the expression or activity of one or more immune activity inhibitory factors selected from the group consisting of LGALS9 (Gal-9), CLEC4G (LSECtin), PD-L1, and HHLA2 of the stem cell, or cells differentiated or derived from stem cells.

The artificially engineered stem cells of the present disclosure, cells differentiated or derived therefrom, or artificially engineered cells differentiated or derived from stem cells exhibit increased expression or activity of one or more immune activity inhibitory factors selected from the group consisting of LGALS9 (Gal-9), CLEC4G (LSECtin), PD-L1, and HHLA2 to inhibit immune rejection when transplanting into a mammal with a disease or condition, and may be used as a composition for cell transplantation or biological tissue regeneration to treat various diseases.

In one embodiment of the present invention, the artificially engineered stem cells of the present disclosure may be differentiated or cultured into various cell types, including human vascular endothelial cells, pancreatic cells, osteocytes, chondrocytes, cardiomyocytes, muscle cells, epidermal cells, fibroblasts, nerve cells, hepatocytes, enterocytes, stomach cells, skin cells, fat cells, blood cells, immune cells, cell spheroids, and organoids and may be used as a composition for cell transplantation or tissue regeneration to restore damaged cells or tissues.

In one embodiment of the present invention, the biological tissue may be a damaged tissue selected from a tissue in which ulcers or bedsores have formed, brain tissue damaged by cell degeneration, brain tissue defected by surgical manipulation, brain tissue damaged by traumatic brain disease, brain tissue damaged by inflammatory brain disease, damaged bone tissue, damaged periodontal tissue, tissue damaged by central nervous system disease, and a tissue damaged by intractable dermatitis, and biological tissue regeneration may be restoration of damaged tissue, epidermal regeneration, reproduction of glands or hair follicles, microvascular formation in dermal tissue, wound healing, correction of soft tissue defects, bone healing, or bone regeneration, cartilage regeneration, or the like, but is not limited thereto.

In one embodiment of the present invention, the composition for cell transplantation or biological tissue regeneration may be a cell therapeutic composition, a gene therapeutic composition, a tissue engineering therapeutic composition, an immunotherapeutic composition, or a composition for the prevention or treatment of cancer.

As used herein, "cell therapeutic agent" refers to a medicine (US FDA regulations) used for the purposes of treatment, diagnosis, and prevention with cells and tissues prepared through isolation, culture, and special engineering from an individual, and refers to a medicine used for treatment, diagnosis, and prevention purposes through a series of actions such as changing the biological characteristics of cells by ex vivo proliferation selection or other methods of living autologous, allogeneic, or xenogeneic cells to restore cell or tissue function.

As used herein, "gene therapeutic agent" refers to a medicine administered to affect the expression of genetic material and containing cells into which genetic material has been modified or introduced.

In one embodiment of the present invention, the disease or condition may be one disease selected from the group consisting of myocardial infarction, heart failure, ischemic cardiomyopathy, myocarditis, ischemic enteritis, inflammatory bowel disease, ulcerative colitis, Crohn's disease, acute cholecystitis, acute cholangitis, chronic cholecystitis, acute pancreatitis, chronic pancreatitis, acute nephritis, chronic nephritis, acute renal failure, chronic renal failure, pneumonia, interstitial pneumonia, idiopathic interstitial pneumonia, exfoliative interstitial pneumonia, acute interstitial pneumonia, non-specific interstitial pneumonia, druginduced lung disease, acute respiratory distress syndrome, chronic obstructive pulmonary disease, cerebral palsy syndrome including pediatric cerebral palsy, amyotrophic lateral sclerosis (ALS), polyneuropathy, spinal muscular atrophy, acute transverse myelitis, stroke, multiple sclerosis, peripheral artery disease (PAD), thromboangiitis obliterans (Buerger's disease), Kawasaki disease (KD), graft versus host disease (GVHD), Parkinson's disease, dementia, Alzheimer's disease, Huntington's disease, amyotrophic lateral sclerosis, memory decline, myasthenia gravis, progressive supranuclear palsy, multiple system atrophy, essential tremor, cortico-basal degeneration, diffuse Lewy body disease, and Pick's disease in a mammal, and the present disclosure may be used to treat and prevent these diseases, is are not limited thereto.

The route of administration of a composition for cell transplantation or biological tissue regeneration of the present disclosure may be administered via any general route as long as the desired tissue may be reached. The route may be administered parenterally, for example, intraperitoneally, intravenously, intramuscularly, subcutaneously, or intradermally, but is not limited thereto.

The composition for cell transplantation or biological tissue regeneration may be formulated in a suitable form with a pharmaceutical carrier commonly used in cell therapy. The term "pharmaceutically acceptable" refers to a composition that is physiologically acceptable and does not usually cause allergic responses such as gastrointestinal disorders, dizziness, or similar responses when administered to humans. Pharmaceutically acceptable carriers include, for example, water, suitable oils, saline solutions, carriers for parenteral administration such as aqueous glucose and glycol, and may further include stabilizers and preservatives. The suitable stabilizer include antioxidants such as sodium bisulfite, sodium sulfite, or ascorbic acid. Suitable preservative include benzalkonium chloride, methylparaben or propyl-paraben, and chlorobutanol. As for other pharmaceutically acceptable carriers, those described in the following literature may be referred to (Remington's Pharmaceutical Sciences, 19th ed., Mack Publishing Company, Easton, PA, 1995). In addition, the composition may be administered by any device that allows the cell therapeutic agent to move to target cells.

The composition for cell transplantation or biological tissue regeneration of the present disclosure may include a therapeutically effective amount of cells for the treatment of disease. The term "therapeutically effective amount" refers to an amount of an active ingredient or pharmaceutical composition that induces a biological or medical response in a tissue system, animal, or human, as believed by a researcher, veterinarian, physician or other clinician, and includes an amount that lead to alleviation of the symptoms of the disease or disorder being treated.

It is obvious to those skilled in the art that the cells included in the composition of the present disclosure will vary depending on a desired effect. Therefore, an optimal cell content may be easily determined by those skilled in the art, and may be adjusted depending on a variety of factors, including the type of disease, the severity of the disease, the content of other ingredients contained in the composition, the type of formulation, and the patient's age, weight, general health, gender and diet, administration time, administration route and secretion rate of the composition, treatment period, and concurrently used medications. Considering all of the above factors, it is important to include a minimum amount of the maximum effect without side effects. For example, the daily dosage of the stem cells of the present disclosure is 1.0×10⁵ to 1.0×10³⁰ cells/kg of body weight, preferably 1.0×10¹⁰ to 1.0×10²⁰ cells/kg of body weight, and may be administered once or in several divided doses. However, it should be understood that the actual dosage of the active ingredient should be determined in light of various related factors such as the disease to be treated, the severity of the disease, the route of administration, the patient's weight, age, and gender, and therefore, the dosage does not limit the scope of the present disclosure in any way.

In addition, a composition including the cells of the present disclosure as an active ingredient in the treatment method for the present disclosure may be administered through routes in the related art such as rectal, intravenous therapy (i.v.), intraarterial, intraperitoneal, intramuscular, intrasternal, transdermal, topical, intraocular or intradermal route. The present disclosure provides a method of treatment including administering a therapeutically effective amount of the artificially engineered stem cells or cells differentiated or derived therefrom to a mammal with a disease or condition. As used herein, the term "mammal" refers to a mammal that is the subject of treatment, observation or experiment, preferably a human.

The present disclosure provides a cell therapy method using stem cells, including: administering a therapeutically effective amount of the artificially engineered stem cells, cells differentiated or derived therefrom, or artificially engineered cells differentiated or derived from stem cells, to a mammal with a disease or condition.

In one embodiment of the present invention, in this method, immune rejection may be suppressed and a state of immune tolerance can be achieved when used as a cell therapeutic agent in an autologous or allogeneic environment by administering artificially engineered stem cells, cells differentiated or derived from artificially engineered stem cells, or artificially engineered cells differentiated or derived from stem cells with increase in expression or activity levels of one or more immune activity inhibitory factors selected from the group consisting of LGALS9 (Gal-9), CLEC4G (LSECtin), PD-L1, and HHLA2.

Hereinafter, the present disclosure will be described in more detail through examples.

These examples are only for illustrating the present disclosure in more detail, and it will be apparent to those skilled in the art that the scope of the present disclosure is not limited by these examples.

### Example 1: Preparation of iPSC (induced pluripotent stem cell) cells with gene inserted (knocked-in) into AAVS1 site

### Cell culture

iPSCs were cultured according to the manufacturer's manual. The cells were cultured using TeSR^{™}-E8^{™} (STEMCELL Technologies) medium in a culture dish coated with Vitronectin XF^{™} (STEMCELL Technologies) for 1 hour at RT (room temperature).

### Insertion of genes (Knock-In, KI)

A complex of guide RNA (sgRNA, SEQ ID NO: 1: gtcaccaatcctgtccctag) and Cas9 protein targeting a specific AAVS1 site, and LGALS9 (Gal-9), CLEC4G (LSECtin), and HHLA2 gene donor was introduced into iPSCs by electroporation (Amaxa 4D nucleofector) to insert a gene (Knock-In, KI).

The complex of sgRNA, Cas9 protein, and gene donor was electroporated with 4×10⁵ iPSCs treated with 20 ul primary P3 buffer using the nucleofector program (CA-137) according to the manufacturer's (Lonza, 4D nucleofector) protocol.

### Experimental Example 1: Confirmation of gene knock-in in iPSC (induced pluripotent stem cell) cells

It was confirmed through PCR whether the gene was inserted into iPSCs (induced pluripotent stem cell) cells in which the gene was inserted (knocked-in) into the AAVS1 site prepared in Example 1.

### PCR (Polymerase chain reaction)

After confirming the base sequence, the gene primers listed in Table 1 below were used, and the PCR reaction was finally extended at 72 °C for 5 minutes to stabilize the PCR product after repeating the processes of first denaturation at 98 °C for 1 minute, followed by denaturation at 98 °C for 20 seconds, annealing at 62°C for 30 seconds, and extension at 72°C for 3 minutes a total of 40 times.

**[Table 1]**

| Primer name | Sequence |
|---|---|
| AAVS1_CT+CAG-ALC3-F2 | 5'-ATTCCCCTTCGACCTACTCT-3' (SEQ ID NO: 2) |
| AAVS1_CT+CAG-ALC3-R2 | 5'-CCGTAAATACTCCACCCATTG-3' (SEQ ID NO: 3) |
| AAVS1_CT+CAG-ALC3-F3 | 5'-ctgaagcaaACATGGACAGG-3' (SEQ ID NO: 4) |
| AAVS1_CT+CAG-ALC3-R3 | 5'-gataccccgaaGAGTGAGTT-3' (SEQ ID NO: 5) |
| Gal9_CLEC4G-KI-F1 | 5'-gcctttcatcaccaccattc-3' (SEQ ID NO: 6) |
| Gal19_CLEC4G-KI-R1 | 5'-agtctccgacctcctccttca-3' (SEQ ID NO: 7) |
| CLEC4G-HHLA2-KI-F1 | 5'-tctctctcagcttcagccact-3' (SEQ ID NO: 8) |
| CLEC4G-HHLA2-KI-R1 | 5'-cccacctttagcaccacttt-3' (SEQ ID NO: 9) |

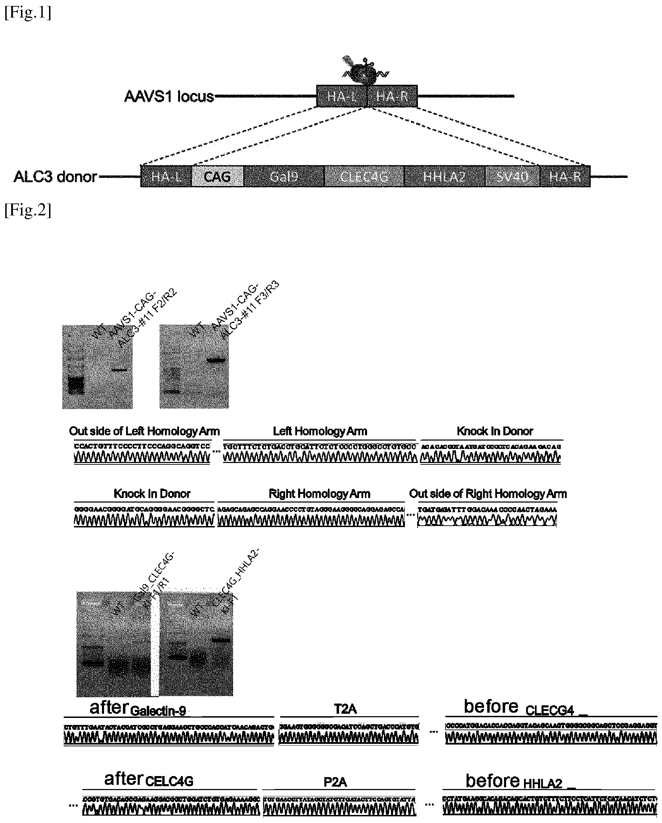

FIG. 2 shows the results of PCR reaction from cell DNA and the results of Sanger sequencing using the PCR product obtained above to select multiple single clones from iPSCs in which three genes prepared in Example 1 were knocked-in (KI), SEQ ID NOs: 10 to 13 are sequence information confirmed from PCR reaction, and as shown in FIG. 2, a specific size band of the inserted gene was confirmed in the KI-iPSCs (AAVS1-ALC3#11) single clone compared to WT-iPSCs without gene knock-in (KI).

### Experimental Example 2: Confirmation of mRNA expression in gene inserted (KI) cells

Changes in mRNA levels of the three genes knocked-in in Example 1 were confirmed as follows.

### Real time PCR (qRT-PCR)

mRNA was extracted from iPSCs using the RNeasy mini kit (Qiagen) according to the manufacturer's protocol. Thereafter, 100 ng of mRNA was reverse transcribed using a cDNA reverse transcription kit (Thermo Fisher Scientific). qRT-PCR was performed using 100 ng of PowerUp^{™} SYBR^{™} Green Master Mix using QuantStudio 3 (Thermo Fisher Scientific) according to the manufacturer's protocol. Gene expression levels were calculated using CT values, and GAPDH was used as an endogenous control.

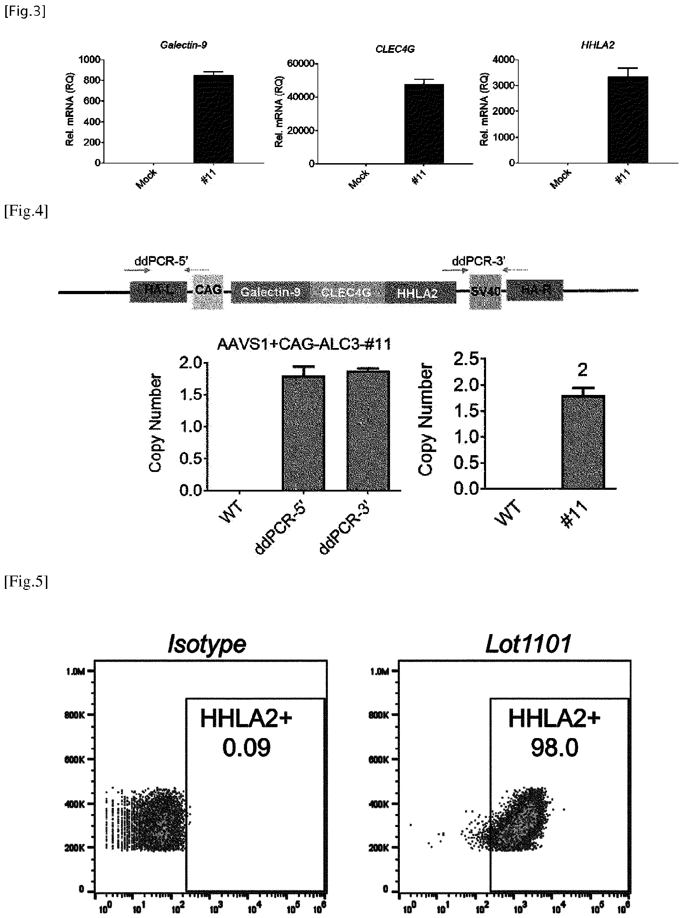

FIG. 3 shows the results of confirming changes in mRNA levels for the three genes knocked-in in Example 1 using real-time PCR. As shown in FIG. 3, it was confirmed that the mRNA levels of Galectin-9, CLEC4G, and HHLA2 were all higher compared to Mock (WT).

### Experimental Example 3: Confirmation of knocked-in gene copy number

ddPCR was performed as follows to confirm the copy number of the gene knocked-in in Example 1.

### ddPCR

Cultured cells were washed twice with phosphate buffer saline (PBS), then cells were removed using 0.05% trypsin-EDTA and centrifuged at 1,000 rpm for 5 minutes. After extracting gDNA from cells, restriction enzyme treatment was performed. The QX200 ddPCR Supermix for probes (Biorad), forward primer (100pmole/ul), reverse primer (100 pmole/ul), taqman probe (10 pmole/ul) shown in Tables 2 and 3 below were taken out at room temperature and prepared. The ddPCR master mix was prepared according to the Biorad manual. The prepared master mix and restriction enzyme-treated template gDNA were plated into a ddPCR 96 well plate. After sealing the plate with the PX1 PCR plate sealer (Biorad), it was mounted on the QX200 Auto DG droplet digital PCR system to generate droplets. After completion of droplet generation, PCR amplification was performed according to the manual protocol. After completion of PCR amplification, the fluorescence value was read and analyzed using the QX200 Droplet reader.

**[Table 2]**

| Primer name | Sequence |
|---|---|
| SV40_quant_F1 | 5'-ggtttgtccaaactcatcaatgt-3' (SEQ ID NO: 14) |
| HA-R_quant_R1 | 5'-ggtgggggttagacccaa-3' (SEQ ID NO: 15) |
| HHLA2_SV40_F1 | 5'-gatgttgtgtccctcctgg-3' (SEQ ID NO: 16) |
| HHLA2_SV40_R1 | 5'-ggacaaaccacaactagaatgcag-3' (SEQ ID NO: 17) |
| AAVS1_HA-L_F2 | 5'-accttatattcccagggccg-3' (SEQ ID NO: 18) |
| AAVS1_HA-L_R2 | 5'-ggctatgaactaatgaccccgt- 3' (SEQ ID NO: 19) |

**[Table 3]**

| Probe name | Sequence |
|---|---|
| SV40-HA-R_probe_3 | 5'-CAGCTTTCTTGTACAAAGTGGGGACAGG-3' (SEQ ID NO: 20) |
| HHLA2_SV40_probe | 5'-tcccagtgcacccgataatggc-3' (SEQ ID NO: 21) |
| AAVS1_HA-L_Probe2 | 5'-tgtcccctccaccccacagtg-3' (SEQ ID NO: 22) |

FIG. 4 is a graph showing the results of confirming the copy number of genes knocked-in (KI) using the ddPCR method. It was confirmed that gene 2 copies were knocked-in (KI) in the KI-iPSC clone.

### Experimental Example 4: Measurement of expression level of knock-in (KI) gene through flow cytometry

To confirm transgene expression of knocked-in (KI) genes, the AAVS1-ALC3#11 clone prepared in Example 1 was analyzed using flow cytometry.

### Flow cytometer analysis

Cultured cells (UC-MSC) were washed twice with phosphate buffer saline (PBS), then cells were removed using 0.05% trypsin-EDTA and centrifuged at 1,000 rpm for 5 minutes. Cells were suspended in 100 ul of FACS staining buffer, mixed with antibodies, reacted at 4 °C for 1 h, washed twice with PBS, and suspended in 500 ul of PBS for FACS analysis.

FIG. 5 shows the results of measuring the HHLA expression level of the AAVS1-ALC3#11 clone prepared in Example 1 through flow cytometry in Experimental Example 4, and 98% of HHLA2+ was confirmed in AAVS1-ALC3#11 Clone compared to the isotype.

### Experimental Example 5: Confirmation of immunogenicity

To evaluate the immunogenicity of induced pluripotent stem cells (iPSCs) cells into which the gene was inserted (knocked-in) prepared in Example 1 (KI-iPSC), and endothelial cell (EC) differentiated from iPSCs into which each gene was inserted (knocked-in) using the lentivirus disclosed in FIG. 8, WT and KI-iPSCs were differentiated into endothelial cells (ECs) as follows. Differentiated endothelial cells were co-cultured with immune cells to confirm immunogenicity.

### Endothelial cell differentiation

Human ESCs were seeded in 6-well plates (Thermo Fisher Scientific) and cultured for 1 day in TeSR^{™}-E8^{™} medium (STEMCELL Technologies) containing 10 mM ROCK inhibitor, Y-27632 (Tocris). The next day, the medium was replaced with N2B27 medium containing 46.9% (v/v) DMEM/F12 (Gibco), 50% (v/v) Neurobasal medium (Gibco), 0.97% N2 (Gibco), 1.94% B27 (Gibco), 0.97% β-mercaptoethanol (Gibco) ), 8 µM CHIR99021 (Cayman Chemical), and 25 ng/ml BMP-4 (Peprotech), and then the cells were incubated for 3 days. The medium was then replaced with StemPro-34 SFM (Thermo Fisher Scientific) supplemented with 100 ng/ml vascular endothelial growth factor (Peprotech), 50 ng/ml basic fibroblast growth factor (Peprotech), and 2 µM forskolin (Abcam), and the cells were incubated for 2 days. Cell populations were then enriched for CD144+ cells using MACS cell separation (Miltenyi Biotec). CD144+ cells were plated and maintained on Matrigel-coated plates containing endothelial cell growth medium (EGM^{™}-2blletKit^{™}) (Lonza) for at least 7 days for further differentiation. The endothelial cells were analyzed by flow cytometer analysis using anti-CD31 antibody (1:25, Clone WM-59, eBioscience).

FIG. 6A shows the results of flow cytometry using an anti-CD31 antibody after differentiating wild-type (WT) iPSCs and iPSCs with knocked-in (KI) three genes prepared in Example 1 into endothelial cells (ECs), and it was observed that 98% or more of cells expressed CD31+, an EC marker.

FIG. 6B shows the results of flow cytometry after differentiating wild-type (WT) iPSCs and iPSCs with knocked-in (KI) three genes prepared in Example 1 into endothelial cells (ECs), and HLA-ABC antigens were confirmed to appear on the surface of EC cells differentiated from iPSCs, and the level was observed to increase further after treatment with interferon gamma at a concentration of 100ng/ml for 48 hours.

### Cytotoxicity assay (in vitro fluorometric cytotoxicity assay to measure NK cell effects)

The effect of NK cells on KI clones (target cells) and K562 cells (ATCC) was assessed by flow cytometry. Target cells were labeled with CellTrace Violet (Thermo Fisher Scientific) for 15 minutes at room temperature and then incubated with NK cells at various ratios in a 5% CO₂ incubator at 37 °C.

To measure the number of apoptotic target cells, washed cells were stained with 7-amino-actinomycin (7-AAD, BD Bioscience) according to the manufacturer's instructions and analyzed using Attune Flow Cytometers (Thermo Fisher Scientific).

FIG. 7A is a graph showing the results of confirming cytotoxicity by culturing K562 cells, endothelial cells differentiated from wild-type (WT) iPSCs (WT-iPSC_EC), and endothelial cells differentiated from iPSCs in which the three genes prepared in Example 1 were knocked in (KI-iPSC EC) with NK cells, which are effector cells, at various ratios. Apoptotic cells were detected with 7-AAD+ cells, and it was confirmed that K562 tumor cells that do not express HLA and endothelial cells differentiated from wild-type (WT) iPSCs (WT-iPSC_EC) were significantly vulnerable to NK cell cytotoxicity.

FIG. 7B is a graph showing the results of evaluating the cytotoxicity of NK cells in endothelial cells differentiated from wild-type (WT) iPSCs expressing 98% or more of HLA-ABC (WT-iPSC_EC, FIG. 6B) and endothelial cells differentiated from iPSCs in which the three genes prepared in Example 1 were knocked in (KI-iPSC EC). Referring to FIG. 7B, as a result of measuring 7-AAD+ cells by co-culturing effector cells (NK cells):target cells (EC, endothelial cells) at a ratio of 1:1, It was confirmed that KI-iPSC_EC had a lower percentage of apoptotic cells compared to WT-iPSC_EC, and as the cell ratio of effector cells (NK cells):target cells (EC, endothelial cells) increased, the percentage of 7-AAD+ cells increased.

### Production of overexpressing EC cells differentiated from iPSC using lentivirus

FIG. 8 is a diagram showing an example of a lentivirus for inserting each gene of LGALS9 (Gal-9)/CLEC4G (LSECtin)/HHLA-2 (SEQ ID NO: 23), LGALS9 (Gal-9) (SEQ ID NO: 24), CLEC4G (LSECtin) (SEQ ID NO: 25), LGALS9 (Gal-9)/CLEC4G (LSECtin) (SEQ ID NO: 26), and LGALS9 (Gal-9)/ CLEC4G (LSECtin)/PD-L1 (SEQ ID NO: 27). At this time, the gene can be inserted into the target sequence on the EC cell genome.

The inserted genes are shown in Tables 4 to 8 below.

**[Table 4]**

| ALC3(SEQ ID NO: 23) |
|---|
| tgctttctctgacctgcattctctcccctgggcctgtgccgctttctgtctgcagcttgtggcctgggtcacctctacggctggcccagatccttccctgccgcctccttcaggtt ccgtcttcctccactccctcttccccttgctctctgctgtgttgctgcccaaggatgctctttccggagcacttccttctcggcgctgcaccacgtgatgtcctctgagcggatcc tccccgtgtctgggtcctctccgggcatctctcctccctcacccaaccccatgccgtcttcactcgctgggttcccttttccttctccttctggggcctgtgccatctctcgtttctt aggatggccttctccgacggatgtctcccttgcgtcccgcctccccttcttgtaggcctgcatcatcaccgtttttctggacaaccccaaagtaccccgtctccctggctttagc cacctctccatcctcttgctttctttgcctggacaccccgttctcctgtggattcgggtcacctctcactcctttcatttgggcagctcccctaccccccttacctctctagtctgtgc tagctcttccagccccctgtcatggcatcttccaggggtccgagagctcagctagtcttcttcctccaacccgggcccctatgtccacttcaggacagcatgtttgctgcctcc agggatcctgtgtccccgagctgggaccaccttatattcccagggccggttaatgtggctctggttctgggtacttttatctgtcccctccaccccacagtggggccacta(H A-L) |
| tgtgcgcgaggggagcgcggccgggggcggtgccccgcggtgcggggggggctgcgaggggaacaaaggctgcgtgcggggtgtgtgcgtgggggggtgagca gggggtgtgggcgcgtcggtcgggctgcaaccccccctgcacccccctccccgagttgctgagcacggcccggcttcgggtgcggggctccgtacggggcgtggcg cggggctcgccgtgccgggcggggggtggcggcaggtgggggtgccgggcggggcggggccgcctcgggccggggagggctcgggggaggggcgcggcggc ccccggagcgccggcggctgtcgaggcgcggcgagccgcagccattgccttttatggtaatcgtgcgagagggcgcagggacttcctttgtcccaaatctgtgcggagc cgaaatctgggaggcgccgccgcaccccctctagcgggcgcggggcgaagcggtgcggcgccggcaggaaggaaatgggcggggagggccttcgtgcgtcgccg cgccgccgtccccttctccctctccagcctcggggctgtccgcggggggacggctgccttcgggggggacggggcagggcggggttcggcttctggcgtgtgaccgg cggctctagagcctctgctaaccatgttcatgccttcttctttttcctacagctcctgggcaacgtgctggttattgtgctgtctcatcattttggcaaagaattc(CAG) |
| atccacacagtgcatgtagaaccgagccaagaaacagcttcccataacaaaggcttatggattttggtgccctctgcgattttggcagcttttctgctgatttggagcgtaaaat gttgcagagcccagctagaagccaggaggagcagacaccctgctgatggagcccaacaagaaagatgttgtgtccctcctggtgagcgctgtcccagtgcacccgataa tggcgaagaaaatgtgcctctttcaggaaaagta (Gal-9/CLEC4G/HHLA2) |
| tagaacttgtttattgcagcttataatggttacaaataaagcaatagcatcacaaatttcacaaataaagcatttttttcactgcattctagttgtggtttgtccaaactcatcaatgtat cttagtcgacagtactaagcttacccagctttcttgtacaaagtgg(SV40) |
| Ggacaggattggtgacagaaaagccccatccttaggcctcctccttcctagtctcctgatattgggtctaacccccacctcctgttaggcagattccttatctggtgacacacc cccatttcctggagccatctctctccttgccagaacctctaaggtttgcttacgatggagccagagaggatcctgggagggagagcttggcagggggtgggagggaaggg ggggatgcgtgacctgcccggttctcagtggccaccctgcgctaccctctcccagaacctgagctgctctgacgcggccgtctggtgcgtttcactgatcctggtgctgca gcttccttacacttcccaagaggagaagcagtttggaaaaacaaaatcagaataagttggtcctgagttctaactttggctcttcacctttctagtccccaatttatattgttcctcc gtgcgtcagttttacctgtgagataaggccagtagccagccccgtcctggcagggctgtggtgaggaggggggtgtccgtgtggaaaactccctttgtgagaatggtgcgt cctaggtgttcaccaggtcgtggccgcctctactccctttctctttctccatccttctttccttaaagagtccccagtgctatctgggacatattcctccgcccagagcagggtcc cgcttccctaaggccctgctctgggcttctgggtttgagtccttggcaagcccaggagaggcgctcaggcttccctgtcccccttcctcgtccaccatctcatgcccctggct ctcctgccccttccctacaggggttcctggctctgctct(HA-R) |

**[Table 5]**

| Gal9(SEQ ID NO: 24) |
|---|
| tgctttctctgacctgcattctctcccctgggcctgtgccgctttctgtctgcagcttgtggcctgggtcacctctacggctggcccagatccttccctgccgcct ccttcaggttccgtcttcctccactccctcttccccttgctctctgctgtgttgctgcccaaggatgctctttccggagcacttccttctcggcgctgcaccacgtg atgtcctctgagcggatcctccccgtgtctgggtcctctccgggcatctctcctccctcacccaaccccatgccgtcttcactcgctgggttcccttttccttctc cttctggggcctgtgccatctctcgtttcttaggatggccttctccgacggatgtctcccttgcgtcccgcctccccttcttgtaggcctgcatcatcaccgtttttc tggacaaccccaaagtaccccgtctccctggctttagccacctctccatcctcttgctttctttgcctggacaccccgttctcctgtggattcgggtcacctctca ctcctttcatttgggcagctcccctaccccccttacctctctagtctgtgctagctcttccagccccctgtcatggcatcttccaggggtccgagagctcagcta gtcttcttcctccaacccgggcccctatgtccacttcaggacagcatgtttgctgcctccagggatcctgtgtccccgagctgggaccaccttatattcccagg gccggttaatgtggctctggttctgggtacttttatctgtcccctccaccccacagtggggccacta(HA-L) |
| tgtgcgcgaggggagcgcggccgggggcggtgccccgcggtgcggggggggctgcgaggggaacaaaggctgcgtgcggggtgtgtgcgtgggg gggtgagcagggggtgtgggcgcgtcggtcgggctgcaaccccccctgcacccccctccccgagttgctgagcacggcccggcttcgggtgcggggc tccgtacggggcgtggcgcggggctcgccgtgccgggcggggggtggcggcaggtgggggtgccgggcggggcggggccgcctcgggccgggg agggctcgggggaggggcgcggcggcccccggagcgccggcggctgtcgaggcgcggcgagccgcagccattgccttttatggtaatcgtgcgaga gggcgcagggacttcctttgtcccaaatctgtgcggagccgaaatctgggaggcgccgccgcaccccctctagcgggcgcggggcgaagcggtgcgg cgccggcaggaaggaaatgggcggggagggccttcgtgcgtcgccgcgccgccgtccccttctccctctccagcctcggggctgtccgcggggggac ggctgccttcgggggggacggggcagggcggggttcggcttctggcgtgtgaccggcggctctagagcctctgctaaccatgttcatgccttcttctttttcc tacagctcctgggcaacgtgctggttattgtgctgtctcatcattttggcaaagaattc(CAG) |
| teaacagactggaagtggggggcgacatecagetgacecatgtgeagaca(Gal-9) |
| tagaacttgtttattgcagcttataatggttacaaataaagcaatagcatcacaaatttcacaaataaagcatttttttcactgcattctagttgtggtttgtccaaact catcaatgtatcttagtcgacagtactaagcttacccagctttcttgtacaaagtgg(SV40) |
| Ggacaggattggtgacagaaaagccccatccttaggcctcctccttcctagtctcctgatattgggtctaacccccacctcctgttaggcagattccttatctg gtgacacacccccatttcctggagccatctctctccttgccagaacctctaaggtttgcttacgatggagccagagaggatcctgggagggagagcttggca gggggtgggagggaagggggggatgcgtgacctgcccggttctcagtggccaccctgcgctaccctctcccagaacctgagctgctctgacgcggccg tctggtgcgtttcactgatcctggtgctgcagcttccttacacttcccaagaggagaagcagtttggaaaaacaaaatcagaataagttggtcctgagttctaac tttggctcttcacctttctagtccccaatttatattgttcctccgtgcgtcagttttacctgtgagataaggccagtagccagccccgtcctggcagggctgtggtg aggaggggggtgtccgtgtggaaaactccctttgtgagaatggtgcgtcctaggtgttcaccaggtcgtggccgcctctactccctttctctttctccatccttct ttccttaaagagtccccagtgctatctgggacatattcctccgcccagagcagggtcccgcttccctaaggccctgctctgggcttctgggtttgagtccttgg caagcccaggagaggcgctcaggcttccctgtcccccttcctcgtccaccatctcatgcccctggctctcctgccccttccctacaggggttcctggctctgc tct(HA-R) |

**[Table 6]**

| CLEC4G(SEQ ID NO: 25) |
|---|
| tgctttctctgacctgcattctctcccctgggcctgtgccgctttctgtctgcagcttgtggcctgggtcacctctacggctggcccagatccttccctgccgcct ccttcaggttccgtcttcctccactccctcttccccttgctctctgctgtgttgctgcccaaggatgctctttccggagcacttccttctcggcgctgcaccacgtg atgtcctctgagcggatcctccccgtgtctgggtcctctccgggcatctctcctccctcacccaaccccatgccgtcttcactcgctgggttcccttttccttctc cttctggggcctgtgccatctctcgtttcttaggatggccttctccgacggatgtctcccttgcgtcccgcctccccttcttgtaggcctgcatcatcaccgtttttc tggacaaccccaaagtaccccgtctccctggctttagccacctctccatcctcttgctttctttgcctggacaccccgttctcctgtggattcgggtcacctctca ctcctttcatttgggcagctcccctaccccccttacctctctagtctgtgctagctcttccagccccctgtcatggcatcttccaggggtccgagagctcagcta gtcttcttcctccaacccgggcccctatgtccacttcaggacagcatgtttgctgcctccagggatcctgtgtccccgagctgggaccaccttatattcccagg gccggttaatgtggctctggttctgggtacttttatctgtcccctccaccccacagtggggccacta(HA-L) |
| tgtgcgcgaggggagcgcggccgggggcggtgccccgcggtgcggggggggctgcgaggggaacaaaggctgcgtgcggggtgtgtgcgtgggg gggtgagcagggggtgtgggcgcgtcggtcgggctgcaaccccccctgcacccccctccccgagttgctgagcacggcccggcttcgggtgcggggc tccgtacggggcgtggcgcggggctcgccgtgccgggcggggggtggcggcaggtgggggtgccgggcggggcggggccgcctcgggccgggg agggctcgggggaggggcgcggcggcccccggagcgccggcggctgtcgaggcgcggcgagccgcagccattgccttttatggtaatcgtgcgaga gggcgcagggacttcctttgtcccaaatctgtgcggagccgaaatctgggaggcgccgccgcaccccctctagcgggcgcggggcgaagcggtgcgg cgccggcaggaaggaaatgggcggggagggccttcgtgcgtcgccgcgccgccgtccccttctccctctccagcctcggggctgtccgcggggggac ggctgccttcgggggggacggggcagggcggggttcggcttctggcgtgtgaccggcggctctagagcctctgctaaccatgttcatgccttcttctttttcc tacagctcctgggcaacgtgctggttattgtgctgtctcatcattttggcaaagaattc(CAG) |
| gccaccatggacaccaccaggtacagcaagtggggcggcagctccgaggaggtccccggagggccctggggacgctgggtgcactggagcaggag acccctcttcttggccctggctgtcctggtcaccacagtcctttgggctgtgattctgagtatcctattgtccaaggcctccacggagcgcgcggcgctgcttg acggccacgacctgctgaggacaaacgcctcgaagcagacggcggcgctgggtgccctgaaggaggaggtcggagactgccacagctgctgctcgg ggacgcaggcgcagctgcagaccacgcgcgcggagcttggggaggcgcaggcgaagctgatggagcaggagagcgccctgcgggaactgcgtga gcgcgtgacccagggcttggctgaagccggcaggggccgtgaggacgtccgcactgagctgttccgggcgctggaggccgtgaggctccagaacaac tcctgcgagccgtgccccacgtcgtggctgtccttcgagggctcctgctactttttctctgtgccaaagacgacgtgggcggcggcgcaggatcactgcgc agatgccagcgcgcacctggtgatcgttgggggcctggatgagcagggcttcctcactcggaacacgcgtggccgtggttactggctgggcctgagggc tgtgcgccatctgggcaaggttcagggctaccagtgggtggacggagtctctctcagcttcagccactggaaccagggagagcccaatgacgcttgggg gcgcgagaactgtgtcatgatgctgcacacggggctgtggaacgacgcaccgtgtgacagcgagaaggacggctggatctgtgagaaaaggcacaact gc(CLEC4G) |
| tagaacttgtttattgcagcttataatggttacaaataaagcaatagcatcacaaatttcacaaataaagcatttttttcactgcattctagttgtggtttgtccaaact catcaatgtatcttagtcgacagtactaagcttacccagctttcttgtacaaagtgg(SV40) |
| Ggacaggattggtgacagaaaagccccatccttaggcctcctccttcctagtctcctgatattgggtctaacccccacctcctgttaggcagattccttatctg gtgacacacccccatttcctggagccatctctctccttgccagaacctctaaggtttgcttacgatggagccagagaggatcctgggagggagagcttggca gggggtgggagggaagggggggatgcgtgacctgcccggttctcagtggccaccctgcgctaccctctcccagaacctgagctgctctgacgcggccg tctggtgcgtttcactgatcctggtgctgcagcttccttacacttcccaagaggagaagcagtttggaaaaacaaaatcagaataagttggtcctgagttctaac tttggctcttcacctttctagtccccaatttatattgttcctccgtgcgtcagttttacctgtgagataaggccagtagccagccccgtcctggcagggctgtggtg aggaggggggtgtccgtgtggaaaactccctttgtgagaatggtgcgtcctaggtgttcaccaggtcgtggccgcctctactccctttctctttctccatccttct ttccttaaagagtccccagtgctatctgggacatattcctccgcccagagcagggtcccgcttccctaaggccctgctctgggcttctgggtttgagtccttgg caagcccaggagaggcgctcaggcttccctgtcccccttcctcgtccaccatctcatgcccctggctctcctgccccttccctacaggggttcctggctctgc tct(HA-R) |

**[Table 7]**

| ALC2(SEQ ID NO: 26) |
|---|
| tgctttctctgacctgcattctctcccctgggcctgtgccgctttctgtctgcagcttgtggcctgggtcacctctacggctggcccagatccttccctgccgcctc cttcaggttccgtcttcctccactccctcttccccttgctctctgctgtgttgctgcccaaggatgctctttccggagcacttccttctcggcgctgcaccacgtgat gtcctctgagcggatcctccccgtgtctgggtcctctccgggcatctctcctccctcacccaaccccatgccgtcttcactcgctgggttcccttttccttctccttc tggggcctgtgccatctctcgtttcttaggatggccttctccgacggatgtctcccttgcgtcccgcctccccttcttgtaggcctgcatcatcaccgtttttctgga caaccccaaagtaccccgtctccctggctttagccacctctccatcctcttgctttctttgcctggacaccccgttctcctgtggattcgggtcacctctcactccttt catttgggcagctcccctaccccccttacctctctagtctgtgctagctcttccagccccctgtcatggcatcttccaggggtccgagagctcagctagtcttcttc ctccaacccgggcccctatgtccacttcaggacagcatgtttgctgcctccagggatcctgtgtccccgagctgggaccaccttatattcccagggccggttaa tgtggctctggttctgggtacttttatctgtcccctccaccccacagtggggccacta(HA-L) |
| tgtgcgcgaggggagcgcggccgggggcggtgccccgcggtgcggggggggctgcgaggggaacaaaggctgcgtgcggggtgtgtgcgtggggg ggtgagcagggggtgtgggcgcgtcggtcgggctgcaaccccccctgcacccccctccccgagttgctgagcacggcccggcttcgggtgcggggctcc gtacggggcgtggcgcggggctcgccgtgccgggcggggggtggcggcaggtgggggtgccgggcggggcggggccgcctcgggccggggaggg ctcgggggaggggcgcggcggcccccggagcgccggcggctgtcgaggcgcggcgagccgcagccattgccttttatggtaatcgtgcgagagggcg cagggacttcctttgtcccaaatctgtgcggagccgaaatctgggaggcgccgccgcaccccctctagcgggcgcggggcgaagcggtgcggcgccggc aggaaggaaatgggcggggagggccttcgtgcgtcgccgcgccgccgtccccttctccctctccagcctcggggctgtccgcggggggacggctgccttc gggggggacggggcagggcggggttcggcttctggcgtgtgaccggcggctctagagcctctgctaaccatgttcatgccttcttctttttcctacagctcctg ggcaacgtgctggttattgtgctgtctcatcattttggcaaagaattc(CAG) |
| tcaacagactggaagtggggggcgacatccagctgacccatgtgcagacaggaagcggagagggcaggggaagtcttctaacatgcggggacgtggag gaaaatcccggccccatggacaccaccaggtacagcaagtggggcggcagctccgaggaggtccccggagggccctggggacgctgggtgcactgga gcaggagacccctcttcttggccctggctgtcctggtcaccacagtcctttgggctgtgattctgagtatcctattgtccaaggcctccacggagcgcgcggcg ctgcttgacggccacgacctgctgaggacaaacgcctcgaagcagacggcggcgctgggtgccctgaaggaggaggtcggagactgccacagctgctg ctcggggacgcaggcgcagctgcagaccacgcgcgcggagcttggggaggcgcaggcgaagctgatggagcaggagagcgccctgcgggaactgcg tgagcgcgtgacccagggcttggctgaagccggcaggggccgtgaggacgtccgcactgagctgttccgggcgctggaggccgtgaggctccagaaca actcctgcgagccgtgccccacgtcgtggctgtccttcgagggctcctgctactttttctctgtgccaaagacgacgtgggcggcggcgcaggatcactgcgc agatgccagcgcgcacctggtgatcgttgggggcctggatgagcagggcttcctcactcggaacacgcgtggccgtggttactggctgggcctgagggctg tgcgccatctgggcaaggttcagggctaccagtgggtggacggagtctctctcagcttcagccactggaaccagggagagcccaatgacgcttgggggcg cgagaactgtgtcatgatgctgcacacggggctgtggaacgacgcaccgtgtgacagcgagaaggacggctggatctgtgagaaaaggcacaactgc (Gal-9/CLEC4G) |
| tagaacttgtttattgcagcttataatggttacaaataaagcaatagcatcacaaatttcacaaataaagcatttttttcactgcattctagttgtggtttgtccaaactc atcaatgtatcttagtcgacagtactaagcttacccagctttcttgtacaaagtgg(SV40) |
| Ggacaggattggtgacagaaaagccccatccttaggcctcctccttcctagtctcctgatattgggtctaacccccacctcctgttaggcagattccttatctggt gacacacccccatttcctggagccatctctctccttgccagaacctctaaggtttgcttacgatggagccagagaggatcctgggagggagagcttggcaggg ggtgggagggaagggggggatgcgtgacctgcccggttctcagtggccaccctgcgctaccctctcccagaacctgagctgctctgacgcggccgtctggt gcgtttcactgatcctggtgctgcagcttccttacacttcccaagaggagaagcagtttggaaaaacaaaatcagaataagttggtcctgagttctaactttggct cttcacctttctagtccccaatttatattgttcctccgtgcgtcagttttacctgtgagataaggccagtagccagccccgtcctggcagggctgtggtgaggagg ggggtgtccgtgtggaaaactccctttgtgagaatggtgcgtcctaggtgttcaccaggtcgtggccgcctctactccctttctctttctccatccttctttccttaaa gagtccccagtgctatctgggacatattcctccgcccagagcagggtcccgcttccctaaggccctgctctgggcttctgggtttgagtccttggcaagcccag gagaggcgctcaggcttccctgtcccccttcctcgtccaccatctcatgcccctggctctcctgccccttccctacaggggttcctggctctgctct(HA-R) |

**[Table 8]**

| ALC2+PDL1(SEQ ID NO: 27) |
|---|
| Gcattctctcccctgggcctgtgccgctttctgtctgcagcttgtggcctgggtcacctctacggctggcccagatccttccctgccgcctccttcaggttccgt cttcctccactccctcttccccttgctctctgctgtgttgctgcccaaggatgctctttccggagcacttccttctcggcgctgcaccacgtgatgtcctctgagc ggatcctccccgtgtctgggtcctctccgggcatctctcctccctcacccaaccccatgccgtcttcactcgctgggttcccttttccttctccttctggggcctgt gccatctctcgtttcttaggatggccttctccgacggatgtctcccttgcgtcccgcctccccttcttgtaggcctgcatcatcaccgtttttctggacaaccccaa agtaccccgtctccctggctttagccacctctccatcctcttgctttctttgcctggacaccccgttctcctgtggattcgggtcacctctcactcctttcatttggg cagctcccctaccccccttacctctctagtctgtgctagctcttccagccccctgtcatggcatcttccaggggtccgagagctcagctagtcttcttcctccaa cccgggcccctatgtccacttcaggacagcatgtttgctgcctccagggatcctgtgtccccgagctgggaccaccttatattcccagggccggttaatgtgg ctctggttctgggtacttttatctgtcccctccaccccacagtggggcaagtttgtacaaaaaagcaggct(HA-L) |
| gggcggtgccccgcggtgcggggggggctgcgaggggaacaaaggctgcgtgcggggtgtgtgcgtgggggggtgagcagggggtgtgggcgcgt cggtcgggctgcaaccccccctgcacccccctccccgagttgctgagcacggcccggcttcgggtgcggggctccgtacggggcgtggcgcggggct cgccgtgccgggcggggggtggcggcaggtgggggtgccgggcggggcggggccgcctcgggccggggagggctcgggggaggggcgcggcg gcccccggagcgccggcggctgtcgaggcgcggcgagccgcagccattgccttttatggtaatcgtgcgagagggcgcagggacttcctttgtcccaaat ctgtgcggagccgaaatctgggaggcgccgccgcaccccctctagcgggcgcggggcgaagcggtgcggcgccggcaggaaggaaatgggcggg gagggccttcgtgcgtcgccgcgccgccgtccccttctccctctccagcctcggggctgtccgcggggggacggctgccttcgggggggacggggcag ggcggggttcggcttctggcgtgtgaccggcggctctagagcctctgctaaccatgttcatgccttcttctttttcctacagctcctgggcaacgtgctggttatt gtgctgtctcatcattttggcaaagaattcacccagctttcttgtacaaagtgg(CAG) |
| aaagcaagcaggagatgttgaagaaaaccccgggcctatgaggatatttgctgtctttatattcatgacctactggcatttgctgaacgcatttactgtcacggt tcccaaggacctatatgtggtagagtatggtagcaatatgacaattgaatgcaaattcccagtagaaaaacaattagacctggctgcactaattgtctattggg aaatggaggataagaacattattcaatttgtgcatggagaggaagacctgaaggttcagcatagtagctacagacagagggcccggctgttgaaggacca gctctccctgggaaatgctgcacttcagatcacagatgtgaaattgcaggatgcaggggtgtaccgctgcatgatcagctatggtggtgccgactacaagcg aattactgtgaaagtcaatgccccatacaacaaaatcaaccaaagaattttggttgtggatccagtcacctctgaacatgaactgacatgtcaggctgagggct accccaaggccgaagtcatctggacaagcagtgaccatcaagtcctgagtggtaagaccaccaccaccaattccaagagagaggagaagcttttcaatgt gaccagcacactgagaatcaacacaacaactaatgagattttctactgcacttttaggagattagatcctgaggaaaaccatacagctgaattggtcatccca gaactacctctggcacatcctccaaatgaaaggactcacttggtaattctgggagccatcttattatgccttggtgtagcactgacattcatcttccgtttaagaa aagggagaatgatggatgtgaaaaaatgtggcatccaagatacaaactcaaagaagcaaagtgatacacatttggaggagacgtaa(Gal9/CLEC4 G/PDL1) |
| tagaacttgtttattgcagcttataatggttacaaataaagcaatagcatcacaaatttcacaaataaagcatttttttcactgcattctagttgtggtttgtccaaact catcaatgtatcttagtcgacagtactaagcttacccagctttcttgtacaaagtgg(SV40) |
| agggacaggattggtgacagaaaagccccatccttaggcctcctccttcctagtctcctgatattgggtctaacccccacctcctgttaggcagattccttatct ggtgacacacccccatttcctggagccatctctctccttgccagaacctctaaggtttgcttacgatggagccagagaggatcctgggagggagagcttggc agggggtgggagggaagggggggatgcgtgacctgcccggttctcagtggccaccctgcgctaccctctcccagaacctgagctgctctgacgcggcc gtctggtgcgtttcactgatcctggtgctgcagcttccttacacttcccaagaggagaagcagtttggaaaaacaaaatcagaataagttggtcctgagttctaa ctttggctcttcacctttctagtccccaatttatattgttcctccgtgcgtcagttttacctgtgagataaggccagtagccagccccgtcctggcagggctgtggt gaggaggggggtgtccgtgtggaaaactccctttgtgagaatggtgcgtcctaggtgttcaccaggtcgtggccgcctctactccctttctctttctccatcctt ctttccttaaagagtccccagtgctatctgggacatattcctccgcccagagcagggtcccgcttccctaaggccctgctctgggcttctgggtttgagtccttg gcaagcccaggagaggcgctcaggcttccctgtcccccttcctcgtccaccatctcatgcccctggctctcctgccccttccctacaggggttcctggctctg ctct(HA-R) |

To stably overexpress the gene, the lentivirus described in FIG. 8 was delivered to EC cells differentiated from WT-iPSCs. The day before delivering the lentivirus, 500,000 cells were cultured in a culture dish, and 24 hours later, the cells were infected with lentivirus particles at an MOI of 100. After infection, medium was replaced with fresh medium and cells were maintained at 37 °C for 48 hours as indicated. The four lentiviruses used were produced by VectorBuilder (Vector Builder Inc, China).

FIG. 9A is a graph showing the results of confirming cytotoxicity by culturing K562 cells, endothelial cells differentiated from wild type (WT) iPSCs (WT-iPSC_EC), endothelial cells (Gal-9_EC) differentiated from iPSCs into which the Gal-9 gene of SEQ ID NO: 24 was inserted, endothelial cells differentiated from iPSCs into which the CLEC4G gene of SEQ ID NO: 25 was inserted (CLEC4G_EC), endothelial cells differentiated from iPSCs into which the Gal-9/CLEC4G gene of SEQ ID NO: 26 was inserted (Gal-9+CLEC4G_EC), and endothelial cells differentiated from iPSCs into which the Gal-9/CLEC4G/PD-L1 gene of SEQ ID NO: 27 was inserted (Gal-9+CLEC4G+ PD-L1_EC) was inserted into endothelial cells differentiated from iPSCs (Gal-9+CLEC4G+ PD-L1_EC) with NK cells, which are effector cells, at various ratios, and FIG. 9B is a graph showing the results of measuring 7-AAD+ cells by co-culturing effector cells (NK cells) and target cells (EC, endothelial cells) at a ratio of 1:1.

As shown in FIG. 9, it was confirmed that endothelial cells in which the Gal-9 gene of SEQ ID NO: 24 was inserted into endothelial cells differentiated from iPSCs (Gal-9_EC), endothelial cells in which the CLEC4G gene of SEQ ID NO: 25 was inserted into endothelial cells differentiated from iPSCs (CLEC4G_EC), endothelial cells in which the Gal-9/CLEC4G gene of SEQ ID NO: 26 was inserted into endothelial cells differentiated from iPSCs (Gal-9+CLEC4G_EC), endothelial cells in which the Gal-9/CLEC4G/PD-L1 gene of SEQ ID NO: 27 was inserted into endothelial cells differentiated from iPSCs (Gal-9+CLEC4G+ PD-L1_EC) had a lower percentage of apoptotic cells than endothelial cells differentiated from wild-type (WT) iPSCs (WT-iPSC_EC), and as the cell ratio of effector cells (NK cells):target cells (EC, endothelial cells) increased, the percentage of 7-AAD+ cells increased.

## Claims

1. Artificially engineered stem cells having artificially engineered genes, in which genes encoding one or more immune activity inhibitory factors selected from the group consisting of LGALS9 (Gal-9), CLEC4G (LSECtin), PD-L1, and HHLA2 are inserted into stem cells genomes.

2. The artificially engineered stem cells of claim 1, wherein the gene sequence of the artificially engineered stem cells is different from the gene sequence of wild-type stem cells, and the artificially engineered stem cells are with increase in the expression or activity of a gene encoding one or more immune activity inhibitory factors selected from the group consisting of LGALS9 (Gal-9), CLEC4G (LSECtin), PD-L1, and HHLA2, or one or more immune activity inhibitory factors selected from the group consisting of LGALS9 (Gal-9), CLEC4G (LSECtin), PD-L1, and HHLA2.

3. The artificially engineered stem cells of claim 1, wherein, in the artificially engineered stem cells, an mRNA transcribed from the artificially engineered gene has a high mRNA expression level or a different sequence compared to the expression level of mRNA transcribed from genes in the wild-type stem cells.

4. The artificially engineered stem cells of claim 1, wherein the stem cells are induced pluripotent stem cells, embryonic stem cells, somatic cell nuclear transfer-PSCs, or adult stem cells.

5. The artificially engineered stem cells of claim 1, wherein the gene encoding one or more immune activity inhibitory factors selected from the group consisting of LGALS9 (Gal-9), CLEC4G (LSECtin), PD-L1, and HHLA2 is knocked in or inserted into the stem cells genomes.

6. Artificially engineered cells differentiated or derived from stem cells, the artificially engineered cells having artificially engineered gene in which genes encoding one or more immune activity inhibitory factors selected from the group consisting of LGALS9 (Gal-9), CLEC4G (LSECtin), PD-L1, and HHLA2 are inserted into genomes of cells differentiated or derived from stem cells.

7. The artificially engineered cell of claim 6, wherein the gene sequence of the artificially engineered cells differentiated or derived from stem cells is different from the gene sequence of wild-type cells, and the artificially engineered cells differentiated or derived from stem cells are with increase in the expression or activity of a gene encoding one or more immune activity inhibitory factors selected from the group consisting of LGALS9 (Gal-9), CLEC4G (LSECtin), PD-L1, and HHLA2, or one or more immune activity inhibitory factors selected from the group consisting of LGALS9 (Gal-9), CLEC4G (LSECtin), PD-L1, and HHLA2.

8. The artificially engineered cells of claim 6, wherein, in the artificially engineered cells differentiated or derived from stem cells, an mRNA transcribed from the artificially engineered gene has a high mRNA expression level or a different sequence compared to the expression level of mRNA transcribed from genes in the wild-type cells.

9. The artificially engineered cells of claim 6, wherein the gene encoding one or more immune activity inhibitory factors selected from the group consisting of LGALS9 (Gal-9), CLEC4G (LSECtin), PD-L1, and HHLA2 is knocked in or inserted into genomes of the cells differentiated or derived from stem cells.

10. The artificially engineered cells of claim 6, wherein the cells differentiated or derived from stem cells are one selected from the group consisting of human vascular endothelial cells, pancreatic cells, osteocytes, chondrocytes, cardiomyocytes, muscle cells, epidermal cells, fibroblasts, nerve cells, hepatocytes, enterocytes, stomach cells, skin cells, fat cells, blood cells, immune cells, cell spheroids, and organoids.

11. The artificially engineered cells of claim 6, wherein the stem cells are induced pluripotent stem cells, embryonic stem cells, somatic cell nuclear transfer-PSCs, or adult stem cells.

12. Cells with increase in expression or activity of one or more immune activity inhibitory factors selected from the group consisting of LGALS9 (Gal-9), CLEC4G (LSECtin), PD-L1, and HHLA2, differentiated or derived from the artificially engineered stem cells of any one of claims 1 to 5.

13. The cells of claim 12, wherein the cells are one selected from the group consisting of human vascular endothelial cells, pancreatic cells, osteocytes, chondrocytes, cardiomyocytes, muscle cells, epidermal cells, fibroblasts, nerve cells, hepatocytes, enterocytes, stomach cells, skin cells, fat cells, blood cells, immune cells, cell spheroids, and organoids.

14. A composition of preparing hypoimmunogenic stem cells or hypoimmunogenic cells differentiated or derived from stem cells, the composition comprising:
a guide nucleic acid capable of targeting a target sequence located in genomes of stem cells or cells differentiated or derived from stem cells, or a nucleic acid encoding the same; and
a gene donor encoding one or more immune activity inhibitory factors selected from the group consisting of LGALS9 (Gal-9), CLEC4G (LSECtin), PD-L1, and HHLA2, or a nucleic acid encoding the same; and
an editor protein or a nucleic acid encoding the same.

15. The composition of claim 14, wherein the composition includes the editor protein and the guide nucleic acid in the form of ribonucleoprotein (RNP).

16. The composition of claim 14, wherein the composition includes a nucleic acid encoding the editor protein and a nucleic acid encoding the guide nucleic acid in the form of one or more vectors.

17. The composition of claim 16, wherein the vector is selected from the group consisting of plasmid, retrovirus, lentivirus, adenovirus, adeno-associated virus, vaccinia virus, poxvirus, and herpes simplex virus.

18. A method of preparing hypoimmunogenic stem cells or hypoimmunogenic cells differentiated or derived from isolated stem cells, the method comprising:
introducing the composition of preparing hypoimmunogenic stem cells or hypoimmunogenic cells differentiated or derived from stem cells of claim 14 into isolated stem cells or cells differentiated or derived from isolated stem cells; and
knocking in a gene encoding one or more immune activity inhibitory factors selected from the group consisting of LGALS9 (Gal-9), CLEC4G (LSECtin), PD-L1, and HHLA2 into genomes of stem cells, or genomes of the cells differentiated or derived from stem cells to increase the expression or activity of one or more immune activity inhibitory factors selected from the group consisting of LGALS9 (Gal-9), CLEC4G (LSECtin), PD-L1, and HHLA2 of the stem cells or of the cells differentiated or derived from isolated stem cells.

19. The method of claim 18, wherein the composition includes a nucleic acid encoding the editor protein and a nucleic acid encoding the guide nucleic acid in the form of one or more vectors.

20. The method of claim 18, wherein a CRISPR/Cas9 complex including Streptococcus pyogenes-derived Cas9 protein and guide RNA that may target a target sequence is contacted with the target sequence located within genome of the stem cell, or of the cell differentiated or derived from stem cells, thereby generating an indel within the target sequence.

21. A composition for cell transplantation or biological tissue regeneration, comprising the artificially engineered stem cell of any one of claims 1 to 5 as an active ingredient.

22. The composition of claim 21, wherein the composition for cell transplantation or biological tissue regeneration is a cell therapeutic composition, a gene therapeutic composition, a tissue engineering therapeutic composition, an immunotherapeutic composition, or a composition for the prevention or treatment of cancer.

23. A composition for cell transplantation or biological tissue regeneration, comprising, as an active ingredient, artificially engineered cells differentiated or derived from stem cells of any one of claims 6 to 11.

24. The composition of claim 23, wherein the composition for cell transplantation or biological tissue regeneration is a cell therapeutic composition, a gene therapeutic composition, a tissue engineering therapeutic composition, an immunotherapeutic composition, or a composition for the prevention or treatment of cancer.

25. A composition for cell transplantation or biological tissue regeneration, the composition comprising, as an active ingredient, cells with increase in expression or activity of one or more immune activity inhibitory factors selected from the group consisting of LGALS9 (Gal-9), CLEC4G (LSECtin), PD-L1, and HHLA2, differentiated or derived from the artificially engineered stem cells of any one of claims 1 to 5.

26. The composition of claim 25, wherein the cells are one selected from the group consisting of human vascular endothelial cells, osteocytes, chondrocytes, cardiomyocytes, muscle cells, epidermal cells, fibroblasts, nerve cells, hepatocytes, enterocytes, stomach cells, skin cells, fat cells, blood cells, immune cells, cell spheroids, and organoids.

27. The composition of claim 25, wherein the composition for cell transplantation or biological tissue regeneration is a cell therapeutic composition, a gene therapeutic composition, a tissue engineering therapeutic composition, an immunotherapeutic composition, or a composition for the prevention or treatment of cancer.
